(19) Europäisches Patentamt
European Patent Office
Office européen des brevets

(11) EP 1 255 724 B1

(12) # EUROPÄISCHE PATENTSCHRIFT

(45) Veröffentlichungstag und Bekanntmachung des Hinweises auf die Patenterteilung:
**30.08.2006 Patentblatt 2006/35**

(21) Anmeldenummer: **00977604.8**

(22) Anmeldetag: **01.12.2000**

(51) Int Cl.:
***C07C 211/38*** *(2006.01)* ***C07C 217/56*** *(2006.01)*
***A61K 31/137*** *(2006.01)*

(86) Internationale Anmeldenummer:
**PCT/EP2000/012107**

(87) Internationale Veröffentlichungsnummer:
**WO 2001/044164 (21.06.2001 Gazette 2001/25)**

(54) **SUBSTITUIERTE NORBORNYLAMINO-DERIVATE, VERFAHREN ZU IHRER HERSTELLUNG, IHRE VERWENDUNG ALS MEDIKAMENT ODER DIAGNOSTIKUM SOWIE SIE ENTHALTENDES MEDIKAMENT**

SUBSTITUTED NORBORNYLAMINO DERIVATIVES, METHOD FOR THE PRODUCTION THEREOF, USE THEREOF AS A MEDICAMENT OR A DIAGNOSTIC REAGENT AND MEDICAMENTS CONTAINING SAID COMPOUNDS

DERIVES NORBORNYLAMINO SUBSTITUES, LEUR PROCEDE DE PREPARATION, LEUR UTILISATION COMME PRODUIT PHARMACEUTIQUE OU DE DIAGNOSTIC, AINSI QU'UN MEDICAMENT QUI LES CONTIENT

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **14.12.1999 DE 19960204**

(43) Veröffentlichungstag der Anmeldung:
**13.11.2002 Patentblatt 2002/46**

(73) Patentinhaber: **Sanofi-Aventis Deutschland GmbH**
**65929 Frankfurt am Main (DE)**

(72) Erfinder:
- **HEINELT, Uwe**
**65187 Wiesbaden (DE)**
- **LANG, Hans-Jochen**
**65719 Hofheim (DE)**
- **KLEEMANN, Heinz-Werner**
**65474 Bischofsheim (DE)**
- **SCHWARK, Jan-Robert**
**65779 Kelkheim (DE)**
- **WIRTH, Klaus**
**65830 Kriftel (DE)**
- **JANSEN, Hans-Willi**
**65527 Niedernhausen (DE)**

(56) Entgegenhaltungen:
**EP-A- 0 825 178**
**WO-A-96/40151**
**US-A- 4 024 274**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft substituierte Norbornylamino- Derivate mit exo-konfiguriertem. Stickstoff und endo-anelliertem Fünf-, Sechs- oder Siebenring der Formel I und mit exo-konfiguriertem Stickstoff und exo-anelliertem Fünf-, Sechs- oder Siebenring der Formel I a,

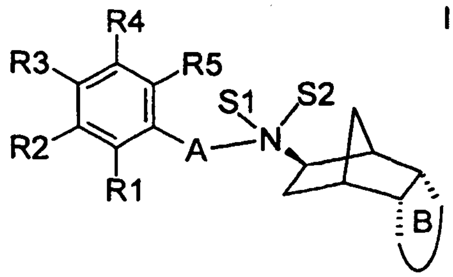
I

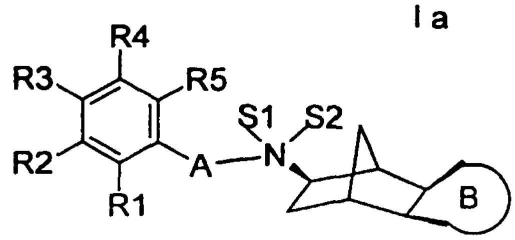
I a

worin bedeuten:

A $(C_1-C_4)$- Alkylen;
S1 ein freies Elektronenpaar oder $(C_1-C_4)$- Alkyl;
S2 $(C_1-C_4)$- Alkyl oder H;
wobei, wenn S1 und S2 Alkyl bedeuten, $X^-$ in der resultierenden Gruppierung [$-N^+$(S1 S2)- $X^-$] einem pharmakologisch akzeptablem Anion oder Trifluoracetat entspricht;
B ein gesättigter oder ungesättigter Kohlenstoff-Fünf-, Sechs- oder Siebenring, der mit Oxo, Hydroxy, $(C_1-C_4)$-Alkoxy und $(C_1-C_4)$- Alkyl einfach oder mehrfach unabhängig voneinander substituiert sein kann;

und

R1, R2, R3, R4 und R5 unabhängig voneinander H, OH, F, Cl, Br, I, CN, $NO_2$, Amidino, $-CO_2R(11)$, -CONR(11)R(12), $-SO_rR(11)$,$-SO_SNR(11)R(12)$, $(C_1-C_4)$-Alkyl, $(C_1-C_4)$-Alkoxy, $(C_1-C_4)$-Alkoxy-$(C_1-C_4)$-alkyl, $(C_1-C_4)$-Alkoxy-$(C_1-C_4)$-alkyloxy, Hydroxy-$(C_1-C_4)$- alkyl, $(C_3-C_7)$- Cycloalkoxy oder Phenyloxy,
wobei Phenyl unsubstituiert ist oder substituiert mit bis zu drei Substituenten, die unabhängig voneinander sind, ausgewählt aus der Gruppe bestehend aus F, Cl, Br und Methoxy;
Amino, $(C_1-C_4)$- Alkylamino, Di-$(C_1-C_4)$- alkylamino, Amino-$(C_1-C_4)$- alkyl, Di-$(C_1-C_4)$- alkylamino-$(C_1-C_4)$- alkyl, $(C_1-C_4)$-Alkylamino-$(C_1-C_4)$- alkyl, wobei die Wasserstoffatome in den Alkylresten ganz oder teilweise durch Fluor substituiert sein können;
R11 und R12 unabhängig voneinander H oder $(C_1-C_4)$- Alkyl,
wobei die Wasserstoffatome in den Alkylresten ganz oder teilweise durch Fluor substituiert sein können;
r 0, 1 oder 2;
s 1 oder 2;oder
R1 und R2, R2 und R3, R3 und R4 oder R4 und R5 jeweils gemeinsam eine Gruppe $-O-(CH_2)_n-O-$;
n 1 oder 2;und
die jeweils verbleibenden Reste R1, R2, R3, R4 oder R5
unabhängig voneinander H, OH, F, Cl, Br, I, CN, $NO_2$, Amidino, $-CO_2R(11)$, - CONR(11)R(12),$-SO_rR(11)$,$-SO_SNR(11)R(12)$,$(C_1-C_4)$-Alkyl,$(C_1-C_4)$-Alkoxy,$(C_1-C_4)$-Alkoxy-$(C_1-C_4)$-alkyl, $(C_3-C_7)$- Cycloalkoxy, Hydroxy-$(C_1-C_4)$-alkyl, Amino, $(C_1-C_4)$- Alkylamino, Di-$(C_1-C_4)$-alkylamino, Amino-$(C_1-C_4)$- alkyl, Di-$(C_1-C_4)$-alkylamino-$(C_1-C_4)$- alkyl, $(C_1-C_4)$-Alkylamino-$(C_1-C_4)$- alkyl,
wobei die Wasserstoffatome in den Alkylresten ganz oder teilweise durch Fluor substituiert sein können;
R11 und R12 unabhängig voneinander H oder $(C_1-C_4)$- Alkyl,
wobei die Wasserstoffatome in den Alkylresten ganz oder teilweise durch Fluor substituiert sein können;
r 0, 1 oder 2;

s 1 oder 2;

wobei Benzyl-(octahydro-4,7-methano-inden-5-yl)-amin ausgenommen ist,
sowie deren pharmazeutisch verträgliche Salze oder Trifluoracetate.

Bevorzugt sind Verbindungen mit exo-konfiguriertem Stickstoff und endo-anelliertem Fünf- oder Sechsring der Formel I und mit exo-konfiguriertem Stickstoff und exo-anelliertem Fünf- oder Sechsring der Formel I a, in der bedeuten:

| | |
|---|---|
| A | ($C_1$-$C_2$)- Alkylen |
| S1 | freies Elektronenpaar oder Methyl, |
| S2 | H; |
| B | ein gesättigter oder ungesättigter Kohlenstoff-Fünf- oder Sechsring; |
| R1, R2, R3, R4 und R5 | unabhängig voneinander H, Amino, Hydroxymethyl, OH, Methoxy, F, Cl, Br oder Iod; |

oder

R2 und R3 gemeinsam -O-$CH_2$-O-;

und
die verbleibenden Reste R1, R4 und R5
unabhängig voneinander H, OH, F, Cl, Br, I, CN, $NO_2$, ($C_1$-$C_2$)- Alkoxy, Amino, ($C_1$-$C_2$)-Alkylamino oder Di-($C_1$-$C_2$)-alkylamino,
wobei die Wasserstoffatome in den Alkylresten ganz oder teilweise durch Fluor substituiert sein können;
wobei Benzyl-(octahydro-4,7-methano-inden-5-yl)-amin ausgenommen ist,
sowie deren pharmazeutisch verträgliche Salze oder Trifluoracetate.

Besonders bevorzugt sind Verbindungen mit exo-konfiguriertem Stickstoff und endo-anelliertem Fünf- oder Sechsring der Formel I und mit exo-konfiguriertem Stickstoff und exo-anelliertem Fünf- oder Sechsring der Formel I a, in der bedeuten:

| | |
|---|---|
| A | ($C_1$-$C_2$)-Alkylen; |
| S1 | freies Elektronenpaar; |
| S2 | H; |
| B | ein gesättigter oder ungesättigter Kohlenstoff-Fünf- oder Sechsring; |
| R1, R3 und R5 | Wasserstoff; |

und R2 und R4
unabhängig voneinander H, Methoxy, F oder Cl;
oder

R2 und R3 gemeinsam -O-$CH_2$-O-;

und

R1, R4 und R5 Wasserstoff;

wobei Benzyl-(octahydro-4,7-methano-inden-5-yl)-amin ausgenommen ist,
sowie deren pharmazeutisch verträgliche Salze oder Trifluoracetate.

Ganz besonders bevorzugt sind die folgenden Verbindungen, mit exo-konfiguriertem Stickstoff und endo-anelliertem Fünf- oder Sechsring der Formel I und mit exo-konfiguriertem Stickstoff und exo-anelliertem Fünfring der Formel I a :

exo/endo-(3-Chlor-benzyl)-(octahydro-4,7-methano-inden-5-yl)-amin,
exo/endo-(3-Fluor-benzyl)-(octahydro-4,7-methano-inden-5-yl)-amin;
exo/endo-Benzo[1,3]dioxol-5-ylmethyl-(octahydro-4,7-methano-inden-5-yl)-amin,
exo/endo-(*rac*)-(3-Methoxy-benzyl)-(octahydro-4,7-methano-inden-5-yl)-amin,

exo/endo-(+)-(3-Methoxy-benzyl)-(octahydro-4,7-methano-inden-5-yl)-amin,
exo/endo-(-)-(3-Methoxy-benzyl)-(octahydro-4,7-methano-inden-5-yl)-amin,
exo/endo-[1-(3-Methoxy-phenyl)-ethyl]-(octahydro-4,7-methano-inden-5-yl)-amin,
exo/endo-(3-Fluor-benzyl)-(3a,4,5,6,7,7a-hexahydro-1H-4,7-methano-inden-5-yl)-amin,
exo/endo-(3-Fluor-benzyl)-(3a,4,5,6,7,7a-hexahydro-3H-4,7-methano-inden-5-yl)-amin,
exo/endo-(3a,4,5,6,7,7a-Hexahydro-1H-4,7-methano-inden-5-yl)-(3-methoxybenzyl)-amin,
exo/endo-(3a,4,5,6,7,7a-Hexahydro-3H-4,7-methano-inden-5-yl)-(3-methoxybenzyl)-amin,
exo/endo-(Decahydro-1,4-methano-naphthalin-2-yl)-(3-methoxy-benzyl)-amin;
exo/endo-(3,5-Difluor-benzyl)-(octahydro-4,7-methano-inden-5-yl)-amin,
exo/exo-(3-Fluor-benzyl)-(octahydro-4,7-methano-inden-5-yl)-amin
und
exo/exo-(3-Methoxy-benzyl)-(octahydro-4,7-methano-inden-5-yl)-amin
sowie deren pharmazeutisch verträgliche Salze oder Trifluoracetate.

Außerordentlich besonders bevorzugt sind die folgenden Verbindungen, mit exo-konfiguriertem Stickstoff und endo-anelliertem Fünf- oder Sechsring der Formel I:

exo/endo-(3-Chlor-benzyl)-(octahydro-4,7-methano-inden-5-yl)-amin,
exo/endo-(3-Fluor-benzyl)-(octahydro-4,7-methano-inden-5-yl)-amin
exo/endo-(3-Fluor-benzyl)-(3a,4,5,6,7,7a-hexahydro-1H-4,7-methano-inden-5-yl)-amin,
exo/endo-(3-Fluor-benzyl)-(3a,4,5,6,7,7a-hexahydro-3H-4,7-methano-inden-5-yl)-amin,
exo/endo-Benzo[1,3]dioxol-5-ylmethyl-(octahydro-4,7-methano-inden-5-yl)-amin,
exo/endo-(rac)-(3-Methoxy-benzyl)-(octahydro-4,7-methano-inden-5-yl)-amin,
exo/endo-(+)-(3-Methoxy-benzyl)-(octahydro-4,7-methano-inden-5-yl)-amin,
exo/endo-(Decahydro-1,4-methano-naphthalin-2-yl)-(3-methoxy-benzyl)-amin
exo/endo-(-)-(3-Methoxy-benzyl)-(octahydro-4,7-methano-inden-5-yl)-amin und
exo/endo-(3,5-Difluorbenzyl)-(octahydro-4,7-methano-inden-5-yl)-amin,
sowie deren pharmazeutisch verträgliche Salze oder Trifluoracetate.

Als Säureadditionssalze kommen dabei Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate, Adipinate, Fumarate, Gluconate, Glutamate, Glycerolphosphate, Maleinate und Pamoate. Diese Gruppe entspricht auch den pharmakologisch akzeptablen Anionen. Aber es kommen auch Trifluoracetate in Frage.

Enthält die Verbindung der Formel I oder I a ein oder mehrere Asymmetriezentren, so können diese sowohl S- als auch R-konfiguriert sein. Die Verbindungen können als optische Isomere, Diastereomere, Racemate oder Gemische derselben vorliegen. Allerdings muß der Aminosubstituent exo-ständig und der Ring endobeziehungsweise exoanelliert sein.

Die genannten Alkyl- oder Alkylen-Reste können sowohl geradkettig als auch verzweigt vorliegen.

Die Erfindung betrifft weiterhin ein Verfahren zur Herstellung der Verbindungen der Formel I oder I a, dadurch gekennzeichnet, daß man

a) eine Verbindung der Formel II oder II a

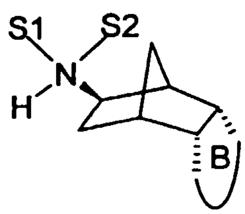

II

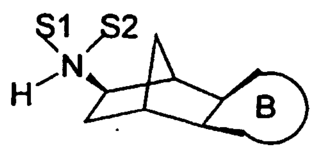

II a

mit einer Verbindung der Formel III in Gegenwart geeigneter Reduktionsmittel und möglicherweise auch Lewis-Säuren direkt zu Verbindungen der Formel I oder I a umsetzt,

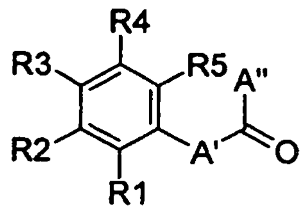

III

worin S1, S2, B, R1, R2, R3, R4 und R5 die oben angegebene Bedeutung besitzen, während unabhängig voneinander A' einer Bindung oder ($C_1$-$C_3$)-Alkyl und A" H oder ($C_1$-$C_3$)- Alkyl entspricht und A' und A" zusammen mit dem Kohlenstoffatom der Carbonylgruppe so viele Kohlenstoffatome repräsentieren wie das oben beschriebene A;
oder
b) das aus Verbindungen der Formeln II oder II a und III gebildete Intermediat der Formel IV oder IV a,

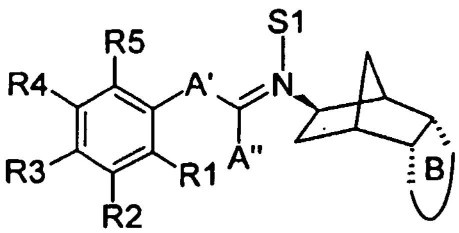

IV

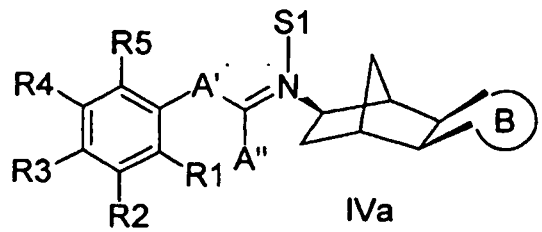

IVa

worin wenn S1 ($C_1$-$C_4$)-Alkyl entspricht, ein Onium-Stickstoff gebildet wird, dem ein Gegenion wie beispielsweise Chlorid oder Tosylat zugeordnet ist, isoliert und dann mit geeigneten Reduktionsmitteln in die Verbindungen der Formel I oder I a überführt,
oder
c) eine Verbindung der Formel II oder II a mit einem Alkylierungsmittel der Formel V,

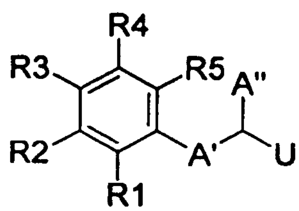

V

in der U für eine nukleophil substituierbare Gruppe steht - wie Chlor, Brom, und Iod sowie Mesylat, Tosylat, Triflat oder eine andere gute Fluchtgruppe-und die anderen Reste wie oben beschrieben definiert sind, aber hier dem Kohlenstoffatom der Carbonylgruppe das Kohlenstoffatom, an das U gebunden ist, entspricht,
vorzugsweise in Gegenwart von nicht nukleophilen Basen wie Diisopropylethylamin umsetzt,
oder
d) Carbonsäureamide der Formel VI oder VI a,

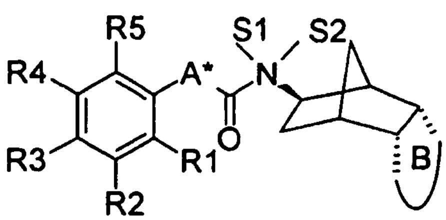

VI

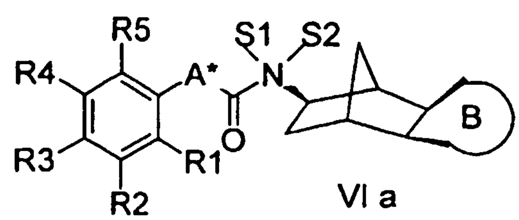

VI a

worin A* einer Bindung oder ($C_1$-$C_3$)-Alkyl entspricht und die anderen Reste, wie oben beschrieben, definiert sind,

zu den entsprechenden Aminen reduziert;

oder

e) Verbindungen der Formel I oder I a, in denen S1 einem freien Elektronenpaar und S2 Wasserstoff entspricht, mit Alkylierungsmitteln der Formel VII,

S*-U VII

worin S* ($C_1$-$C_4$)- Alkyl bedeutet und U oben beschriebene Bedeutung besitzt, mono- oder dialkyliert, so daß aus dieser Umsetzung tertiäre Amine oder quartäre Ammoniumsalze hervorgehen;

oder

f) einen Dicyclopentadienylplatin-Komplex der Formel VIII

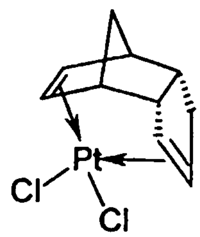

VIII

mit Aminen vom Typ der Formel IX,

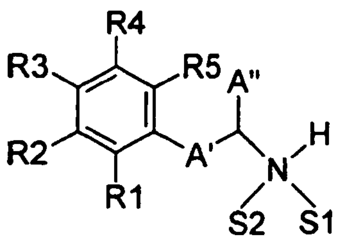

IX

worin S1, S2, R1, R2, R3, R4 und R5 die oben angegebene Bedeutung besitzen, während unabhängig voneinander A' einer Bindung oder ($C_1$-$C_3$)-Alkyl und A" H oder ($C_1$-$C_3$)-Alkyl entspricht und A' und A" zusammen mit dem Kohlenstoffatom, an welches das Stickstoffatom gebunden ist, so viele Kohlenstoffatome repräsentieren wie das oben beschriebene A,

umsetzt und anschließend das gebildete Zwischenprodukt zu Verbindungen der Formel I reduziert (J. K. Stille, D. B. Fox JACS 1970 (92), 1274),

und daß man gegebenenfalls in das pharmazeutisch verträgliche Salz oder Trifluoracetat überführt.

Die US- Patentschrift 4. 024 274 (Hoe 74/F018) beschreibt Norbomylamine ähnlichen Strukturtyps, aber unbekannter räumlicher Struktur mit guter diuretischer und saluretischer Wirksamkeit. WO 96/40151 offenbart die Verwendung von Squalamin zur Herstellung eines Arzneimittels zur Hemmung von NHE.

Im Screening fielen aus der Vielzahl der dortigen Patentbeispiele einige Verbindungen dieses Strukturtyps überraschend als potente Inhibitoren des Natrium-Protonen-Austauschers, Subtyp 3 (NHE3), auf. Die potenteste Verbindung wurde daraufhin auf ihre salidiuretische Wirksamkeit untersucht und überraschenderweise konnte keine salidiuretische Wirkung nachgewiesen werden, so daß ein Zusammenhang zwischen NHE3-Aktivität und Salidiurese nicht aufgezeigt werden konnte.

Da die räumliche Struktur des Tricyclus nicht bekannt war, bestand die Wahl zwischen vier möglichen Enantiomerenpaaren, also insgesamt acht räumlich verschiedenen Strukturen. Bei diesen Enantiomerenpaaren zeigte sich, daß nur zwei Paare eine potente NHE3-inhibierende Wirkung aufweisen, während die beiden anderen Enantiomerenpaare kaum NHE3-blockierende Eigenschaften zeigen. Die Aufklärung der aktivsten Struktur durch Röntgenstrukturanalyse offenbarte, daß es sich bei dem am stärksten NHE3-aktiven Enantiomerenpaar um Verbindungen mit definierter exo-Konfiguration für den Stickstoff sowie definiert endo-anelliertem Fünfring handelt. Das etwas weniger aktive Enantiomerenpaar besitzt die definierte exo-Konfiguration für den Stickstoff sowie einen definiert exo-anellierten Fünfring. Die beiden verbliebenen Enantiomerenpaare mit definierter endo/exobeziehungsweise endo/endo-Konfiguration zeigen

kaum NHE3-inhibierende Wirkung.

Überraschend war weiterhin, daß die definierten getrennten Enantiomere einer der Beispielverbindungen, die die definierte exo-Konfiguration für den Stickstoff sowie den definiert endo-anellierten Fünfring aufweisen, beide ähnlich aktiv am NHE3 sind. Aufgrund ihrer spiegelbildlichen räumlichen Anordnung war hier ein deutlicher Aktivitätsunterschied zu erwarten.

Gegenüber den bekannten Inhibitoren des Natrium-Protonen-Austauschers, Subtyp 3 nach EP-OS 825 178 (HOE 96/F226), die relativ polare Strukturen repräsentieren und dem Acylguanidintyp entsprechen (J.-R. Schwark et al. Eur. J. Physiol (1998) 436:797), handelt es sich bei den erfindungsgemäßen Verbindungen um überraschend lipophile Substanzen, die nicht vom Acylguanidintyp sind und die völlig neuartige Strukturen für die Inhibierung des NHE3 repräsentieren. Es handelt sich unseren Recherchen zufolge nach den soeben genannten Acylguanidinen und dem verzögert wirkenden Squalamin (M. Donowitz et al. Am. J. Physiol. 276 (Cell Physiol. 45): C136-C144; Aktivität wird nach einer Stunde beobachtet) erst um die dritte Stoffklasse von NHE3-Inhibitoren, die bislang bekannt wurde. Gegenüber den obigen bekannten NHE3-Inhibitoren zeichnen sie sich durch überlegene Membrangängigkeit und unverzögerten Wirkeintritt aus.

Der NHE3 wird im Körper verschiedener Spezies bevorzugt in der Galle, dem Darm und in der Niere gefunden (Larry Fliegel et al, Biochem. Cell. Biol. 76: 735 - 741, 1998), ließ sich aber auch im Gehirn nachweisen (E. Ma et al. Neuroscience 79: 591 - 603).

Die erfindungsgemäßen Verbindungen der Formel I oder I a eignen sich zur Verwendung als Antihypertensiva für die Behandlung der primären und sekundären Hypertonie.

Außerdem können die Verbindungen alleine oder in Verbindung mit NHE-Inhibitoren anderer Subtypspezifität akut oder chronisch sauerstoffmangelversorgte Organe durch Verringerung oder Verhinderung ischämisch induzierter Schäden schützen.

Sie eignen sich somit als Arzneimittel z.B. für operative Eingriffe (z.B. bei OrganTransplantationen von Niere und Leber, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können) oder akutes und chronisches Nierenversagen. Besonders vorteilhaft lassen sich ischämisch induzierte Schäden am Darm vermeiden.

Entsprechend ihrer protektiven Wirkung gegen ischämisch induzierte Schäden sind die Verbindungen potenziell auch als Arzneimittel zur Behandlung von Ischämien des Nervensystems, insbesondere des ZNS, geeignet, wobei sie z. B. zur Behandlung des Schlaganfalls oder des Hirnödems geeignet sind. Darüber hinaus eignen sich die erfindungsgemäßen Verbindungen der Formel I oder I a ebenfalls zur Behandlung von Formen des Schocks, wie beispielsweise des allergischen, cardiogenen, hypovolämischen und des bakteriellen Schocks.

Weiterhin induzieren die Verbindungen eine Verbesserung des Atemantriebes und werden deshalb zur Behandlung von Atmungszuständen bei folgenden klinischen Zuständen und Krankheiten herangezogen: Gestörter zentraler Atemantrieb (z. B. zentrale Schlafapnoen, plötzlicher Kindstod, postoperative Hypoxie), muskulärbedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.

Zusätzlich erhöhen die Verbindungen den Muskeltonus der oberen Atemwege, so daß das Schnarchen unterdrückt wird.

Eine Kombination eines NHE-Inhibitors mit einem Carboanhydrase-Hemmer (z. B. Acetazolamid), wobei letzterer eine metabolische Azidose herbeiführt und dadurch bereits die Atmungstätigkeit steigert, erweist sich als vorteilhaft durch verstärkte Wirkung und verminderten Wirkstoffeinsatz.

Es hat sich gezeigt, daß die erfindungsgemäßen Verbindungen eine milde abführende Wirkung besitzen und demzufolge vorteilhaft als Abführmittel oder bei drohender Darmverstopfung verwendet werden können, wobei die Verhinderung der mit Verstopfungen im Darmbereich einhergehenden ischämischen Schäden besonders vorteilhaft ist.

Weiterhin besteht die Möglichkeit der Gallenstein-Bildung vorzubeugen.

Darüber hinaus können die erfindungsgemäßen Verbindungen der Formel I oder I a eine inhibierende Wirkung auf die Proliferationen von Zellen, beispielsweise der Fibroblasten-Zellproliferation und der Proliferation der glatten Gefäßmuskelzellen ausüben. Deshalb kommen die Verbindungen der Formel I oder I a als wertvolle Therapeutika für Krankheiten in Frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, endotheliale Dysfunktion, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters, der bei zahlreichen Erkrankungen (essentielle Hypertonie, Atherosklerose, Diabetes usw.) auch in solchen Zellen erhöht ist, die Messungen leicht zugänglich sind wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw. Darüber hinaus sind die Verbindungen der Formel I oder I a für die präventive Therapie zur Verhinderung der Genese des Bluthochdrucks, beispielweise der essentiellen Hypertonie, geeignet.

Es wurde außerdem gefunden, daß NHE-Inhibitoren eine günstige Beeinflussung der Serumlipoproteine zeigen. Es ist allgemein anerkannt, daß für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter SerumLipoproteine eine außerordentliche Bedeutung zu. Die erfindungsgemäßen Verbindungen können daher zur Prophylaxe und zur Regression von atherosklerotischen Veränderungen herangezogen werden, indem sie einen kausalen Risikofaktor ausschalten. Mit diesem Schutz der Gefäße gegen das Syndrom der endothelialen Dysfunktion sind Verbindungen der Formel I oder I a wertvolle Arzneimittel zur Prävention und zur Behandlung koronarer Gefäßspasmen, der Atherogenese und der Atherosklerose, der linksventrikulären Hypertrophie und der dilatierten Kardiomyopathie, und thrombotischer Erkrankungen.

Die genannten Verbindungen finden deshalb vorteilhaft Verwendung zur Herstellung eines Medikaments zur Prävention und Behandlung von Schlafapnoen und muskulär bedingter Atemstörungen; zur Herstellung eines Medikaments zur Prävention und Behandlung des Schnarchens; zur Herstellung eines Medikaments zur Blutdrucksenkung; zur Herstellung eines Medikaments mit abführender Wirkung zur Prävention und Behandlung intestinaler Verstopfungen; zur Herstellung eines Medikaments zur Prävention und Behandlung von Erkrankungen, die durch Ischämie und Reperfusion von zentralen und peripheren Organen ausgelöst werden wie das akute Nierenversagen, der Schlaganfall, endogene Schockzustände, Darmerkrankungen etc.; zur Herstellung eines Medikaments zur Behandlung von Hypercholesterinämie; zur Herstellung eines Medikaments zur Prävention der Atherogenese und der Atherosklerose; zur Herstellung eines Medikaments zur Prävention und Behandlung von Krankheiten, die durch erhöhte Cholesterinspiegel ausgelöst werden; zur Herstellung eines Medikaments zur Prävention und Behandlung von Krankheiten, die durch endotheliale Dysfunktion ausgelöst werden; zur Herstellung eines Medikaments zur Behandlung des Befalls durch Ektoparasiten; zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten. Eine Kombination eines NHE-Inhibitors der Formel I oder I a mit einem blutfettspiegelsenkenden Wirkstoff, bevorzugt mit einem HMG-CoA-Reduktaseinhibitor (z. B. Lovastatin oder Pravastatin), wobei letzterer eine hypolipidämische Wirkung herbeiführt und dadurch die hypolipidämischen Eigenschaften des NHE-Inhibitors der Formel I oder I a steigert, erweist sich als günstige Kombination mit verstärkter Wirkung und vermindertem Wirkstoffeinsatz.

Beansprucht wird die. Gabe von Natrium-Protonen-Austausch-Hemmern der Formel I oder I a als neuartige Arzneimittel zur Senkung erhöhter Blutfettspiegel, sowie die Kombination von Natrium-Protonen-Austausch-Hemmern mit blutdrucksenkenden und/oder hypolipidämisch wirkenden Arzneimitteln.

Arzneimittel, die eine Verbindung I oder I a enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I oder I a können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I oder I a in einem pharmazeutisch unbedenklichen Lösungsmittel, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer

Konzentration von etwa 0,1 bis 10, insbesondere von etwa 0,3 bis 3 Gew.-%.

Die Dosierung des zu verabreichenden Wirkstoffs der Formel I oder I a und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I oder I a bei einem etwa 75 kg schweren Patienten mindestens 0,001 mg/kg, vorzugsweise 1-10 mg/kg, bis höchstens 100 mg/kg Körpergewicht. Bei akuten Ausbrüchen der Krankheiten können auch noch höhere und vor allem häufigere Dosierungen notwendig sein, z. B. bis zu 4 Einzeldosen pro Tag. Insbesondere bei i. v. Anwendung, etwa bei einem Infarktpatienten auf der Intensivstation können bis zu 200 mg pro Tag notwendig werden.

Experimenteller Teil:

Verwendete Abkürzungen:

| | |
|---|---|
| $CH_2Cl_2$ | Dichlormethan |
| CI | chemische Ionisation |
| DIP | Diisopropylether |
| EE | Ethylacetat |
| ES | Elektrospray |
| HOAc | Essigsäure |
| $H_2O$ | Wasser |
| $H_2O_2$ | Wasserstoffperoxid |
| kp | Siedepunkt |
| MeOH | Methanol |
| $MgSO_4$ | Magnesiumsulfat |

| | |
|---|---|
| mp | Schmelzpunkt |
| MS | Massenspektrum |
| MTB | Metyl-tert.-butylether |
| $NaBH_4$ | Natriumborhydrid |
| $NaHCO_3$ | Natriumhydrogencarbonat |
| NaOH | Natiumhydroxid |
| RT | Raumtemperatur |
| THF | Tetrahydrofuran |
| TFA | Trifluoressigsäure |
| HCl | Salzsäure |

Synthesebeschreibung einiger Amine:

Amin 1)

Synthese des exo/endo-konfigurierten Octahydro-4,7-methano-inden-5-ylamins:

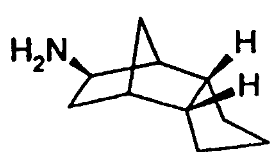

a1) Bis-(6-chlor-3a,4,5,6,7,7a-hexahydro-1H-4,7-methano-inden-5-yl)-diazen N,N'-dioxid und Isomere

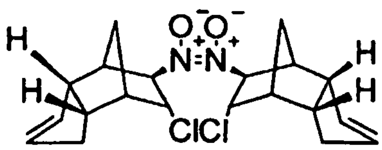

In eine Mischung aus 167g Dicyclopentadien, 160 ml Eisessig und 160 ml Ethanol gab man 167 g iso-Amylnitrit und tropfte sodann unter Rühren bei -10˚C 420 ml einer 15%igen Lösung von Chlorwasserstoff in Ethanol zu. Man rührte weitere 3 Stunden bei Raumtemperatur. Nach Zugabe von 500 ml Diisopropylether rührte man weitere 10 Minuten und filtrierte die Kristalle ab. Nahezu farblose Kristalle, mp. 177-178˚C.

b1) Octahydro-4,7-methano-inden-5-ylamin

Eine Suspension aus 10 g Bis-(6-chlor-3a,4,5,6,7,7a-hexahydro-1H-4,7-methano-inden-5-yl)-diazen N,N'- dioxid, 60 ml Methanol und Raney-Nickel wurde über 10 Stunden bei 100˚C unter 100 atm $H_2$-Druck hydriert. Man filtrierte den Katalysator ab, verdampfte das Lösungsmittel am Rotationsverdampfer unter vermindertem Druck, versetzte den halbkristallinen Rückstand mit Wasser und stellte durch Zugabe von 10 N NaOH stark alkalisch. Nach 3- bis 4-maligem Extrahieren mit Methyl-tert.-butylether und Trocknen der organischen Phasen über Natriumsulfat destillierte man das Lösungsmittel ab und rektifizierte das Öl im Vakuum. $Kp_{5mm}$ 86 - 91˚ C

oder

a2) 3a,4,5,6,7,7a-Hexahydro-1 H-4,7-methano-inden-5-ylamin und 3a,4,5,6,7,7a-Hexahydro-3H-4,7-methano-inden-5-ylamin

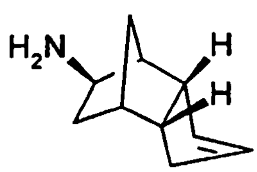

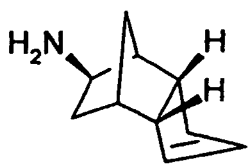

20 g exo-5-Isothiocyanat-5,6-dihydroendodicyclopentadien (Maybridge international) wurden in 60 ml Ameisensäure gelöst und 27 Stunden unter Rückfluß gekocht. Die flüchtigen Bestandteile wurden im Vakuum entfernt, 50 ml einer 20%igen wäßrigen NaOH-Lösung zugegeben und dreimal mit je 100 ml $CH_2Cl_2$ extrahiert. Über $MgSO_4$ wurde getrocknet und das Solvens im Vakuum entfernt. Man erhielt 13,4 g eines blaßgelben Öls.

$R_f$($CH_2Cl_2$/MeOH/HOAc/$N_2O$ 32:8:1:1) = 0,57; MS (ES+): 150 $(M+H)^+$

b2) (3a,4,5,6,7,7a-Hexahydro-1 H-4,7-methano-inden-5-yl)-carbaminsäure-tert-butylester und (3a,4,5,6,7,7a-Hexahydro-3H-4,7-methano-inden-5-yl)-carbaminsäure-tert-butylester

12,8 g eines Gemisches aus 3a,4,5,6,7,7a-Hexahydro-1H-4,7-methano-inden-5-ylamin und 3a,4,5,6,7,7a-Hexahydro-3H-4,7-methano-inden-5-ylamin wurden in 200 ml THF gelöst und bei RT mit einer Lösung von 18,7 g Di-tert-butyl-dicarbonat in 200 ml THF versetzt. Anschließend wurden 12 ml Triethylamin zugetropft und 2 Stunden bei RT gerührt. Die flüchtigen Bestandteile wurden im Vakuum entfernt und der Rückstand an Kieselgel mit DIP chromatographiert. Man erhielt 15 g eines farblosen Öls, das aus n-Heptan kristallisierte; mp 94˚ C.

$R_f$(DIP) = 0.68 MS (Cl+) : 250 $(M+H)^+$

c2) (Octahydro-4,7-methano-inden-5-yl)-carbaminsäure-tert-butylester

500 mg eines Gemisches aus (3a,4,5,6,7,7a-Hexahydro-1H-4,7-methano-inden-5-yl)-carbaminsäure-tert-butylester und (3a,4,5,6,7,7a-Hexahydro-3H-4,7-methano-inden-5-yl)-carbaminsäure-tert-butylester wurden in 20 ml Methanol und 2 ml Essigsäure gelöst und mit Hilfe von 200 mg Pd/C 10% (50% Wasser) unter einer Wasserstoffatmosphäre (1 bar) während 6 Stunden hydriert. Der Katalysator wurde abfiltriert und die flüchtigen Bestandteile im Vakuum entfernt. Man erhielt 470 mg eines harzähnlichen amorphen Feststoffs.

$R_f$(DIP) = 0,70 MS (Cl+) : 252 $(M+H)^+$

d2) Octahydro-4,7-methano-inden-5-ylamin-Trifluoracetat

460 mg (Octahydro-4,7-methano-inden-5-yl)-carbaminsäure-tert-butylester wurden in 5 ml Trifluoressigsäure gelöst und 24 Stunden bei RT gerührt. Anschließend wurden die flüchtigen Bestandteile im Vakuum entfernt, und man erhielt 390 mg eines blaßgelben Schaums.

$R_f$(EE/HEP/MeOH/$CH_2Cl_2$/gesättigte wässrige $NH_3$-Lösung 10:5:5:5:1) = 0,30

MS (Cl+): 152 $(M+H)^+$

oder

a3) Octahydro-4,7-methano-inden-5-ylamin

3,3 g eines Gemisches aus 3a,4,5,6,7,7a-Hexahydro-1 H-4,7-methano-inden-5-ylamin und 3a,4,5,6,7,7a-Hexahydro-3H-4,7-methano-inden-5-ylamin (Beispiel Amin 1, a2) wurden in 30 ml Methanol gelöst und unter Wasserstoffatmosphäre in Gegenwart von 0,5 g Pd/C (10%) reduziert. Nach 4 Stunden wurde der Katalysator abfiltriert und mit Methanol gewaschen. Das Filtrat wurde im Vakuum eingeengt und lieferte 3 g des gewünschten Produkts als Öl.
MS(ES+): 152 $(M+H)^+$

Amin 2)

Synthese des endo/exo-konfigurierten Octahydro-4,7-methano-inden-5-ylamins:

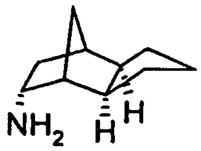

Eine zuvor bei 10°C mit $NH_3$ gesättigte Lösung aus 15 g Tricyclo[5,2,1,$0^{2,6}$]decan-8-on in 60 ml Methanol wurde nach Zugabe von Raney-Nickel im Autoklaven bei 90°C und einem Wasserstoffdruck von 100 bar über 10 Stunden hydriert. Nach Filtration des Katalysators und Destillation des Lösungsmittels unter vermindertem Druck stellte man mit 10 N NaOH stark alkalisch und extrahierte 2 - 3-mal mit Ethylacetat oder mit Diisopropylether. Die vereinigten organischen Phasen wurden getrocknet und im Vakuum fraktioniert destilliert. $K_{P6\text{-}7mm}$ 86 - 88°C.

Amin 3)

Synthese des endo/endo-konfigurierten Octahydro-4,7-methano-inden-5-ylamins:

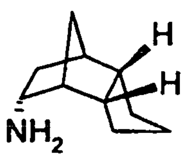

a) 1,3a,4,6,7,7a-Hexahydro-4,7-methano-inden-5-on-oxim 10 g Bis-(6-chlor-3a,4,5,6,7,7a-hexahydro-1H-4,7-methano-inden-5-yl)-diazen N,N'- dioxid aus Amin 1, a1) wurden in 75 ml Isoamylalkohol suspendiert und unter Rühren langsam auf Rückfluß erhitzt. Wenn sich alles gelöst hatte, wurde mit dem Eisbad auf Raumtemperatur abgekühlt, und es wurden 25 ml trockenes Ethanol, 12,5 ml Eisessig und 6 g Zinkstaub zugegeben. Nach einer Stunde Rückfluß wurde abgekühlt, das Zink abfiltriert und das Ethanol abgezogen. Der Rückstand wurde in 300 ml Ether eingerührt und über Nacht stehengelassen. Der Ether wurde dann vom Niederschlag abdekantiert, und dreimal mit Natriumcarbonat-Lösung und zweimal mit Wasser gewaschen. Nach Trocknen über Magnesiumsulfat wurde filtriert und dann das Filtrat eingeengt. Die anschließende Vakuumdestillation lieferte 3,3 g Öl, das direkt weiter umgesetzt wurde.

b) Octahydro-4,7-methano-inden-5-ylamin
2,2 g 1,3a,4,6,7,7a-Hexahydro-4,7-methano-inden-5-on-oxim wurden in 50 ml Methanol gelöst und ca. 10% Raney-Nickel, gelöst in 50% Wasser, zugegeben. Nachdem 20 Stunden bei 100 bar und 100°C hydriert worden war, wurde der Katalysator abfiltriert und das Lösungsmittel abgezogen. Der Rückstand wurde mit Ether und 6 N Natronlauge aufgenommen, die Phasen getrennt, die wäßrige Phase dreimal mit Ether extrahiert, die vereinigten organischen Phasen mit Magnesiumsulfat getrocknet, filtriert und das Filtrat eingeengt. Es blieben 1,8 g eines farblosen Öls zurück, das durch Kugelrohrdestillation gereinigt wurde. Es wurden 0,96 g des gewünschten Amins als Öl erhalten.
MS (CI+): 152,2 $(M+H)^+$

Amin 4)

Synthese des exo/exo- konfigurierten Octahydro-4,7-methano-inden-5-ylamins:

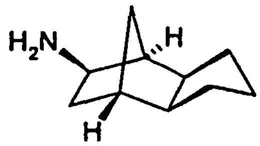

a) Octahydro-4,7-methano-inden-5-ol

25 g Tricyclo[5.2.1.0 (2,6)]decan-8-on (Aldrich) wurden in 100 ml Methanol gelöst und bei Raumtemperatur unter leichter Kühlung und Rühren portionsweise innerhalb von 2 h mit 6,3 g festem Natriumborhydrid versetzt. Anschließend wurde 2 h nachgerührt und über Nacht stehengelassen. Unter Kühlung wurden dann ca. 40 ml 2 N HCl zugetropft, gefolgt von 20 ml Wasser. Das Gemisch wurde eingeengt, der Rückstand mit Essigester versetzt und die Essigesterphase einmal mit Wasser und einmal mit Natriumhydrogencarbonat-Lösung gewaschen. Nach Trocknen mit Magnesiumsulfat wurde die Essigesterphase filtriert und eingeengt. Es blieben 26 g Öl zurück, das durch Vakuumdestillation gereinigt wurde. Es wurden 20,7 g einer öligen Flüssigkeit erhalten ($kp_{0,5}$ 76˚C).

b) 2-(Octahydro-4,7-methano-inden-5-yl)-isoindol-1,3-dion

Zu einer Lösung aus 1,66 g Octahydro-4,7-methano-inden-5-ol, 1,47 g Phthalimid und 2,62 g Triphenylphosphin in 15 ml THF wurden unter Rühren 1,7 g Diethylazodicarboxylat verdünnt mit 5 ml THF gegeben. Nach Stehen über Nacht wurde das Reaktionsgemisch eingedampft, der Rückstand mit Ether verrührt, der Niederschlag abgesaugt und das Filtrat eingeengt. Der Rückstand wird über Kieselgel/Toluol gereinigt. Es werden 1,36 g eines gelben Öls erhalten.
MS (Cl+): 282,2 $(M+H)^+$

c) exo/exo- Octahydro-4,7-methano-inden-5-ylamin

Zu einer Lösung aus 1,12 g 2-(Octahydro-4,7-methano-inden-5-yl)-isoindol-1,3-dion und 15 ml Ethanol wurden 0,4 g Hydrazinhydrat getropft und 2 h bei 65 ˚C gerührt. Anschließend wurde mit konz. HCl auf pH 1-2 gestellt, mit 10 ml Ethanol versetzt, der Niederschlag abfiltriert und das Filtrat eingeengt. Der Rückstand wurde mittels präparativer HPLC an RP-18 mit Acetonitril/Wasser (0,05% Trifluoressigsäure) gereinigt. Nach Gefriertrocknung wurden 567 mg Produkt als Trifluoracetat erhalten. Behandlung mit Natronlauge und Essigester lieferte 322 mg des freien Amins.
MS (Cl+): 152,0 $(M+H)^+$

Beispiele:

Wenn nicht anders beschrieben, handelt es sich bei den hier angeführten Beispielen um Racemate.

Beispiel 1: (exo/endo)-(3-Chlor-benzyl)-(octahydro-4,7-methano-inden-5-yl)-amin-Hydrochlorid

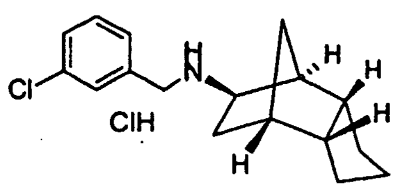

Eine Lösung aus 0,54 g des exo/endo-konfigurierten Octahydro-4,7-methano-inden-5-ylamins (Amin 1) und 0,562 g 3-Chlorbenzaldehyd in 20 ml Toluol werden nach Zugabe einer kleinen katalytischen Menge an p-Toluolsulfonsäure für 5 Stunden zum Sieden erhitzt, und nach dem Stehenlassen über Nacht bei Raumtemperatur wird das Lösungsmittel abdestilliert. Man löst den Rückstand in Methanol und gibt sodann unter Rühren zu der eisgekühlten gelben Lösung in kleinen Portionen 0,181 g Natriumboranat. Man rührt mehrere Stunden bei Raumtemperatur und stellt sodann mit überschüssiger methanolischer Chlorwasserstofflösung stark sauer. Nach kurzem Rühren filtriert man den Niederschlag ab und destilliert im Filtrat das Lösungsmittel ab. Der Rückstand bildet eine farblose bis schwach gelbe kristalline Substanz, mp 241˚C.

Beispiel 2: (exo/endo)-(3-Fluor-benzyl)-(3a,4,5,6,7,7a-hexahydro-1H-4,7-methano-inden-5-yl)-amin und (exo/endo)-(3-Fluor-benzyl)-(3a,4,5,6,7,7a-hexahydro-3H-4,7-methano-inden-5-yl)-amin

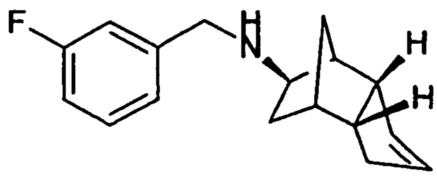

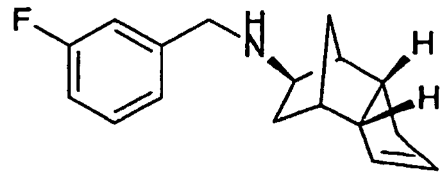

300 mg eines Gemisches aus 3a,4,5,6,7,7a-Hexahydro-1H-4,7-methano-inden-5-ylamin und 3a,4,5,6,7,7a-Hexahydro-3H-4,7-methano-inden-5-ylamin (siehe Amin 1, a2), 315 µl 3-Fluorbenzaldehyd sowie 10 mg p-Toluolsulfonsäure wurden in 5 ml Toluol (wasserfrei) gelöst und 5 Stunden unter Rückfluß gekocht. Anschließend wurden die flüchtigen Bestandteile im Vakuum entfernt, der Rückstand mit 20 ml MeOH aufgenommen, 152 mg $NaBH_4$ zugegeben und 15 Stunden bei RT stehengelassen. Das Reaktionsgemisch wurde dann mit 200 ml EE verdünnt und zweimal mit je 50 ml einer gesättigten wäßrigen $NaHCO_3$-Lösung gewaschen. Über $MgSO_4$ wurde getrocknet und das Solvens im Vakuum entfernt. Präparative HPLC an RP-18 mit Acetonitril/Wasser (Gradient: 5:95 - 95:5) liefert 150 mg eines farblosen Öls.
$R_f$(EE) = 0,40; MS (CI+) : 258 $(M+H)^+$

Beispiel 3: (exo/endo)-(3a,4,5,6,7,7a-Hexahydro-1H-4,7-methano-inden-5-yl)-(3-methoxy-benzyl)- amin und (exo/endo)-(3a,4,5,6,7,7a-Hexahydro-3H-4,7-methano-inden-5-yl)-(3-methoxy-benzyl)-amin

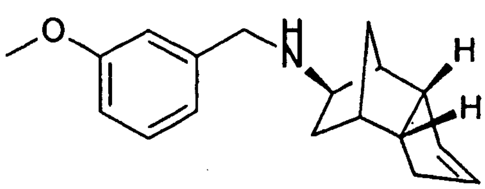

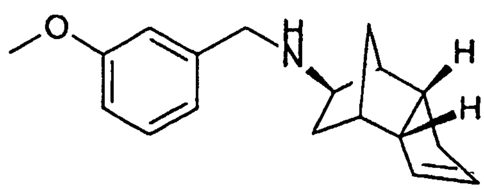

Die Verbindungen des Beispiels 3 wurde analog Beispiel 2) synthetisiert.
$R_f$(EE) = 0,35; MS (CI+) : 270 $(M+H)^+$

Beispiel 4: (exo/endo)-5-(3-Methoxy-benzylamino)-octahydro-4,7-methano-inden-2-ol

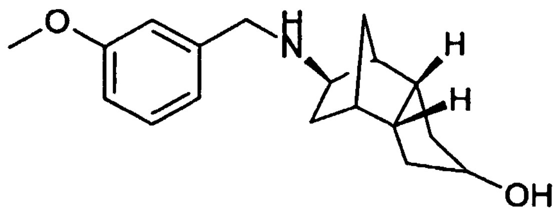

a) (3a,4,5,6,7,7a-Hexahydro-1H-4,7-methano-inden-5-yl)-carbaminsäure-tert-butylester und (3a,4,5,6,7,7a-Hexahydro-3H-4,7-methano-inden-5-yl)-carbaminsäure-tert-butylester

12,8 g eines Gemisches aus 3a,4,5,6,7,7a-Hexahydro-1H-4,7-methano-inden-5-ylamin und 3a,4,5,6,7,7a-Hexahydro-3H-4,7-methano-inden-5-ylamin wurden in 200 ml THF gelöst und bei RT mit einer Lösung von 18,7 g Di-tert.-butyl-dicarbonat in 200 ml THF versetzt. Anschließend wurden 12 ml Triethylamin zugetropft und 2 Stunden bei RT gerührt. Die flüchtigen Bestandteile wurden im Vakuum entfernt. Chromatographie an Kieselgel mit DIP lieferte 15 g eines farblosen Öls. Kristallisation aus n-Heptan lieferte 4,9 g farbloser Kristalle, mp 94°C.
$R_f$(DIP) = 0,68; MS (ES+): 250 $(M+H)^+$

b) (2-Hydroxy-octahydro-4,7-methano-inden-5-yl)-carbaminsäure-tert-butylester

4,87 g eines Gemisches aus (3a,4,5,6,7,7a-Hexahydro-1 H-4,7-methano-inden-5-yl)-carbaminsäure-tert-butylester und (3a,4,5,6,7,7a-Hexahydro-3H-4,7-methano-inden-5-yl)-carbaminsäure-tert-butylester wurden in 30 ml Toluol (wasserfrei) gelöst und bei RT 20 ml einer 2 M Lösung von Boran Dimethylsulfid-Komplex in Toluol zugespritzt. 24 Stunden wurde bei RT gerührt, weitere 10 ml einer 2 M Lösung von Boran Dimethylsulfid-Komplex.in Toluol zugespritzt und nochmals 6 Stunden bei RT gerührt. Die flüchtigen Bestandteile wurden anschließend im Vakuum entfernt, 200 ml $CH_2Cl_2$ und 33 ml einer 3 N wäßrigen NaOH-Lösung zugegeben und langsam mit 7 ml einer 30% wäßrigen $H_2O_2$-Lösung versetzt. 10 Minuten wurde bei RT gerührt und weitere 100 ml einer 3 N wäßrigen NaOH-Lösung sowie 20 ml einer 30% wäßrigen $H_2O_2$-Lösung zugegeben. Weitere 10 Minuten wurde bei RT gerührt und anschließend das Reaktionsgemisch dreimal mit je 200 ml $CH_2Cl_2$ extrahiert. Über $MgSO_4$ wurde getrocknet und das Solvens im Vakuum entfernt. Chromatographie an Kieselgel mit MTB lieferte 2,9 g eines amorphen Feststoffs, der noch mit Regioisomeren verunreinigt war.
$R_f$(MTB) = 0,47; MS (Cl+) : 268 $(M+H)^+$

c) 5-Amino-octahydro-4,7-methano-inden-2-ol-Trifluoracetat

300 mg (2-Hydroxy-octahydro-4,7-methano-inden-5-yl)-carbaminsäure-tert-butylester wurden in 3 ml Trifluoressigsäure gelöst und 30 Minuten bei RT gerührt. Anschließend wurden die flüchtigen Bestandteile im Vakuum entfernt. Man erhielt 340 mg eines harzähnlichen Feststoffs, der so weiter eingesetzt wurde. $R_f$(EE/HEP/MeOH/$CH_2Cl_2$/gesättigte wäßrige $NH_3$-Lösung 10:5:5:5:1) = 0,28; MS (ES+) : 168 $(M+H)^+$

d) 5-(3-Methoxy-benzylamino)-octahydro-4,7-methano-inden-2-ol

309 mg 5-Amino-octahydro-4,7-methano-inden-2-ol-Trifluoracetat und 225 mg 3-Methoxybenzaldehyd wurden in 10 ml Toluol (wasserfrei) gelöst und 5 Stunden unter Rückfluß gekocht. Anschließend wurden die flüchtigen Bestandteile im Vakuum entfernt. Der Rückstand wurde in 10 ml MeOH aufgenommen, mit 208 mg $NaBH_4$ versetzt und 16 Stunden bei RT gerührt. Anschließend wurde mit 100 ml EE verdünnt und zweimal mit je 30 ml einer 10% wäßrigen $NaHCO_3$- Lösung gewaschen. Über $MgSO_4$ wurde getrocknet und das Solvens im Vakuum entfernt. Chromatographie an Kieselgel mit EE/MeOH 2 : 1 lieferte 100 mg eines amorphen Feststoffs.
$R_f$(EE/MeOH 2:1) = 0,20; MS (ES+): 288 $(M+H)^+$

Beispiel 5: *rac*-(exo/endo)-(3-Methoxy-benzyl)-(octahydro-4,7-methano-inden-5-yl)-amin-Hydrochlorid

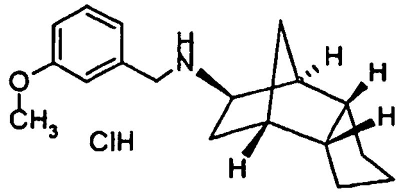

Eine Mischung aus 1,08 g 3-Methoxybenzaldehyd, 1,1 g (exo/endo)-Octahydro-4,7-methano-inden-5-ylamin (Amin1), einer katalytische Menge p-Toluolsulfonsäure und 20 ml wasserfreiem Toluol wurde 3 Stunden unter Rückfluß gekocht, das Toluol unter vermindertem Druck abdestilliert und der Rückstand in 20 ml Methanol gelöst. Zu dieser methanolischen Lösung fügte man in kleinen Portionen unter Kühlung 0,36 g Natriumborhydrid und rührte 18 Stunden bei Raumtemperatur. Nach Zugabe einer Lösung von Chlorwasserstoff in Methanol filtrierte man vom Niederschlag ab und destillierte das Lösungsmittel unter vermindertem Druck ab. Man kochte den Rückstand in Ethanol, filtrierte ab und gab zum Filtrat unter Rühren 150 ml Diethylether. Man stellte mehrere Stunden in den Kühlschrank und filtrierte die kristalline Substanz ab. Farblose Kristalle, mp. 190 - 194˚C.

Beispiel 6: (+)-(exo/endo)-(3-Methoxy-benzyl)-(octahydro-4,7-methano-inden-5-yl)-amin-Hydrochlorid und

(-)-(exo/endo)-(3-Methoxy-benzyl)-(octahydro-4,7-methano-inden-5-yl)-amin-Hydrochlorid

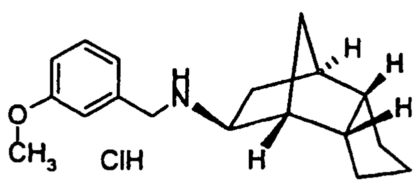

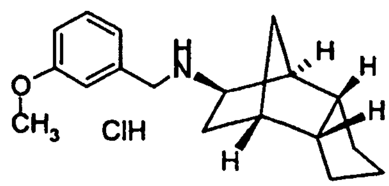

500 mg *rac*-(3-Methoxy-benzyl)-(octahydro-4,7-methano-inden-5-yl)-amin-Hydrochlorid aus Beispiel 5) wurden in mehreren Läufen auf einer präparativen Säule von Diacel Chemicals (CSP-Chiralpak-AS 250x25, 10μ) getrennt. Die Bedingungen waren wie folgt: Fluß: 3 ml/ min, Temperatur: 24˚C, Eluentengemisch: n-Hexan/Ethanol/i-Propanol/TFA 10/1/1/0,1, Wellenlänge: 230 nm.
Nach Gefriertrocknung wurden erhalten:

(+)-Enantiomer: 198 mg, HPLC-Reinheit: 98%
(-)-Enantiomer: 218 mg, HPLC-Reinheit: 99%

Zur Überführung ins Hydrochlorid wurden 75 mg des jeweiligen Enantiomers mit Kaliumcarbonat-Lösung und Ethylacetat versetzt und gut geschüttelt. Nach Phasentrennung wurde die wäßrige Phase noch zweimal mit Ethylacetat extrahiert. Die vereinigten organischen Phasen wurden mit Magnesiumsulfat getrocknet, filtriert und im Vakuum eingeengt. Der Rückstand wurde mit Ethylacetat über 5 g Kieselgel filtriert, das Filtrat eingeengt, der Rückstand mit 2 N Salzsäure versetzt und gefriergetrocknet.
Nach Gefriertrocknung wurden erhalten:

(+)-Enantiomer: 53 mg, Drehwert: +33˚ (Na, 589 nm), MS (ES+): 272,2 $(M+H)^+$
(-)-Enantiomer: 51 mg, Drehwert: -32˚, (Na, 589 nm), MS (ES+): 272,2 $(M+H)^+$

Beispiel 7: (endo/exo)-(3-Methoxy-benzyl)-(octahydro-4,7-methano-inden-5-yl)-amin-Hydrochlorid

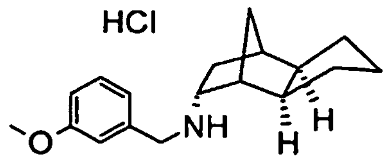

Eine Mischung aus 2,2 g 3-Methoxybenzaldehyd, 40 ml Methanol, 3,3 g (endo/exo)-Octahydro-4,7-methano-inden-5-ylamin (Amin 2) und Raney-Nickelkatalysator wurde im Autoklaven bei 80˚C, 60 bar Wasserstoffdruck über 6 Stunden hydriert. Man löste den Rückstand in Ethylacetat, filtrierte vom Katalysator ab, destillierte das Lösungsmittel unter vermindertem Druck ab, trocknete über Natriumsulfat und vertrieb das Lösungsmittel erneut am Rotavapor. Nach Auflösen des Rückstands in wenig Ethylacetat versetzte man mit einem Überschuß an etherischer Salzsäure, aus der sich unter Rühren ein Niederschlag abschied. Farblose kristalline Substanz (aus Diisopropylether/Methanol) vom mp. 206 - 208˚C.

Beispiel 8: (endo/endo)-(3-Methoxy-benzyl)-(octahydro-4,7-methano-inden-5-yl)-amin-Hydrochlorid

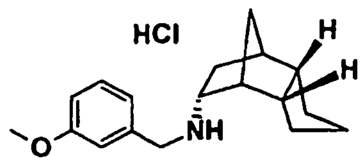

190 mg 3-Methoxybenzaldehyd, 211 mg (endo/endo)-Octahydro-4,7-methano-inden-5-ylamin (Amin 3) und 423 mg Triethylamin wurden unter Feuchtigkeitsausschluß in 5 ml trockenem $CH_2Cl_2$ vorgelegt. Über ein Septum wurden 0,7 ml einer 1 molaren Lösung von Titantetrachlorid in Toluol unter Rühren zugetropft. Nach 18 Stunden bei Raumtemperatur wurden 887 mg Triacetoxyborhydrid zugegeben und eine weitere Stunde gerührt. Anschließend wurden 3 ml 5 N Natriumhydroxid-Lösung und 10 ml Wasser zugesetzt, dann wurde dreimal mit 20 ml Ethylacetat extrahiert, getrocknet, filtriert und im Vakuum eingeengt. Der Rückstand wurde in 2 N Salzsäure gelöst und mit Ether extrahiert. Die wäßrige Phase wurde eingedampft und mittels präparativer HPLC an RP-18 mit Acetonitril/Wasser gereinigt. Die sauberen Fraktionen wurden vereinigt, das Acetonitril am Rotationsverdampfer entfernt, mit Kaliumcarbonat auf pH 11 gestellt, mit $CH_2Cl_2$ extrahiert, die vereinigten Phasen getrocknet und eingeengt. Der Rückstand wurde mit 2 N Salzsäure und etwas Acetonitril aufgenommen und gefriergetrocknet. Es wurden 10 mg des Hydrochlorids als weißer Feststoff erhalten. MS (ES+): 272,2 $(M+H)^+$

Beispiel 9: (exo/exo)-(3-Methoxy-benzyl)-(octahydro-4,7-methano-inden-5-yl)-amin-Hydrochlorid

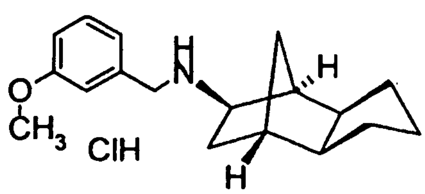

Eine Mischung aus 150 mg 3-Methoxybenzaldehyd, 167 mg (exo/exo)-Octahydro-4,7-methano-inden-5-ylamin (Amin 4), einer katalytischen Menge p-Toluolsulfonsäure und 15 ml wasserfreiem Toluol wurde 3 Stunden unter Rückfluß gekocht, das Toluol unter vermindertem Druck abdestilliert und der Rückstand in 10 ml Methanol gelöst. Zu dieser methanolischen Lösung fügte man unter Eiskühlung in kleinen Portionen 50 mg Natriumborhydrid und ließ auf Raumtemperatur kommen. Nach Zugabe einer Lösung von Chlorwasserstoff in Methanol filtrierte man vom Niederschlag ab und destillierte das Lösungsmittel unter vermindertem Druck ab. Der Rückstand wurde mittels präparativer HPLC an

RP-18 mit Acetonitril/Wasser (0,05% Trifluoressigsäure) gereinigt. Das daraus nach Gefriertrocknung erhaltene Trifluoracetat wurde mittels Natronlauge/Essigester in das freie Amin überführt und dann mit 2 N HCl in das Hydrochlorid überführt. Es wurden 125 mg weißes Produkt erhalten.
MS (Cl+): 272,2 $(M+H)^+$

Beispiel 10: (exo/endo)-(3-Fluor-benzyl)-(octahydro-4,7-methano-inden-5-yl)-amin-Hydrochlorid

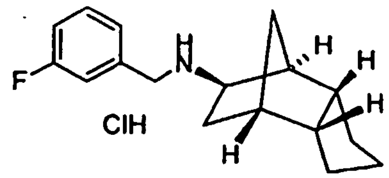

124 mg 3-Fluorbenzaldehyd, 151 mg (exo/endo)-Octahydro-4,7-methano-inden-5-ylamin (Amin 1) und 303 mg Triethylamin wurden unter Feuchtigkeitsausschluß in 10 ml trockenem $CH_2Cl_2$ vorgelegt. Über ein Septum wurden 0,5 ml einer einmolaren Lösung von Titantetrachlorid in Toluol unter Rühren zugetropft. Nach 18 Stunden bei Raumtemperatur wurden 3 ml einer einmolaren Lösung von Natriumcyanoborhydrid in THF zugegeben und weitere 15 min. gerührt. Anschließend wurden 5 ml 5 N Natriumhydroxid-Lösung und 15 ml Wasser zugesetzt, dann wurde dreimal mit 25 ml Ethylacetat extrahiert, getrocknet, filtriert und im Vakuum eingeengt. Der Rückstand wurde über Kieselgel filtriert ($CH_2Cl_2$/Methanol 97:3), erneut zur Trockne gebracht und das Rohprodukt mittels präparativer HPLC an RP-18 mit Acetonitril/Wasser gereinigt. Die sauberen Fraktionen wurden vereinigt, das Acetonitril am Rotationsverdampfer entfernt, mit Kaliumcarbonat auf pH 11 gestellt, dreimal mit Ethylacetat extrahiert und die vereinigten Phasen getrocknet und eingeengt. Der Rückstand wurde mit 2 N Salzsäure und etwas Acetonitril aufgenommen und gefriergetrocknet. Es wurden 144 g mg eines weißen Feststoffs erhalten.
MS (Cl+): 260 $(M+H)^+$

Beispiel 11:(exo/endo)-(3,5-Difluor-benzyl)-(octahydro-4,7-methano-inden-5-yl)-amin-Hydrochlorid

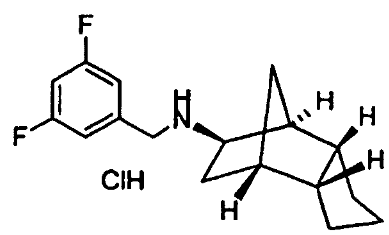

200 mg 3,5-Difluorbenzaldehyd und 151 mg (exo/endo)-Octahydro-4,7-methano-inden-5-ylamin (Amin 1) wurden in 15 ml Toluol (wasserfrei) gelöst, mit 11 mg para-Toluolsulfonsäure versetzt und 3 Stunden unter Rückfluß gekocht. Anschließend wurden die flüchtigen Bestandteile im Vakuum entfernt. Der Rückstand wurde in 10 ml Methanol aufgenommen, unter Eiskühlung und Rühren mit 64 mg $NaBH_4$ versetzt und über Nacht stehengelassen.
Die Lösung wurde mit methanolischer HCl auf pH 1-2 gestellt, der ausgefallene Feststoff abfiltriert und die Lösung eingeengt. Der Rückstand wurde heiß in Ethanol gelöst, filtriert und unter Rühren abgekühlt. Zugabe von Diethylether fällte das Produkt. Es wurde abgesaugt, mit Ether gewaschen und getrocknet. Es wurden 212 mg eines weißen Feststoffs erhalten.
MS (Cl+): 278,3 $(M+H)^+$

Beispiel 12: (exo/endo)-[1-(3-Methoxy-phenyl)-ethyl]-(octahydro-4,7-methano-inden-5-yl)-amin-Hydrochlorid

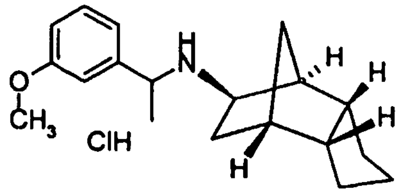

Zu einer Lösung aus 0,75 g 3-Methoxy-acetophenon und 2,7 g (exo/endo)-Octahydro-4,7-methano-inden-5-ylamin (Amin 1) in 15 ml n-Pentan tropfte man bei 5-10°C innerhalb von 10 Minuten eine Mischung aus 0,73 g Titantetrachlorid und 3 ml n-Pentan. Man rührte eine weitere Stunde bei 5-10°C und ließ sodann über Nacht bei Raumtemperatur stehen. Nach Filtration des Niederschlages destillierte man das Lösungsmittel am Rotavapor ab. Nun löste man den Rückstand in 20 ml Methanol und versetzte portionsweise unter Kühlung bei 5-10°C mit 0,96 g Natriumborhydrid. Die Mischung wurde 15 - 20 Stunden bei Raumtemperatur gerührt und dann das Lösungsmittel abdestilliert. Man versetzte den Rückstand mit Wasser, stellte mit Salzsäure sauer und behandelte extrahierend mit Ethylacetat, wobei sich Kristalle abschieden, die abfiltriert und aus wenig Wasser umkristallisiert wurden (mp. 257 - 259°C). Das wäßrige Filtrat stellte man mit 2 N NaOH stark alkalisch, extrahierte mit Ethylacetat und destillierte das Lösungsmittel nach dem Trocknen der organischen Lösung über Natriumsulfat ab. Nach dem Auflösen des Rückstandes in wenig Ethylacetat stellte man mit einer Lösung aus Chlorwasserstoff in Diethylether stark sauer, filtrierte die Kristalle ab und kristallisierte aus wenig Wasser um (mp. 257 - 259°C).

Beispiel 13: (exo/endo)-(3-Brom-benzyl)-(octahydro-4,7-methano-inden-5-yl)-amin-Hydrochlorid

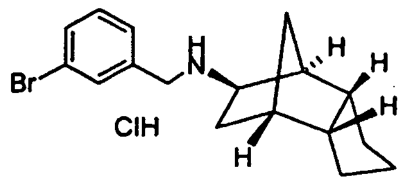

1,9 g 3-Brombenzaldehyd, 1,5 g (exo/endo)-Octahydro-4,7-methano-inden-5-ylamin (Amin 1) und 60 mg p-Toluolsulfonsäure wurden in 180 ml wasserfreiem Toluol gelöst und 5 Stunden unter Rückfluß gekocht. Die flüchtigen Bestandteile wurden im Vakuum entfernt und der Rückstand in 120 ml Methanol gelöst. 530 mg $NaBH_4$ wurden zugegeben und 2 Stunden bei RT gerührt. 18 Stunden wurde bei RT stehen gelassen, anschließend die flüchtigen Bestandteile im Vakuum entfernt. Mit 200 ml einer gesättigten wäßrigen $NaHCO_3$-Lösung wurde aufgenommen und dreimal mit je 200 ml EE extrahiert. Über $MgSO_4$ wurde getrocknet und das Solvens im Vakuum entfernt. Der Rückstand wurde mit 12 ml einer 10 % wässrigen HCl-Lösung aufgenommen und die flüchtigen Bestandteile im Vakuum entfernt. Der Rückstand wurde mit 50 ml EE verrührt und man erhielt 3,0 g des kristallinen Hydrochlorids, mp 248°C.
$R_f$(EE) = 0,44; MS (Cl+) : 320 $(M+H)^+$

Beispiel 14: (exo/endo)-3-[(Octahydro-4,7-methano-inden-5-ylamino)-methyl]-benzoesäure

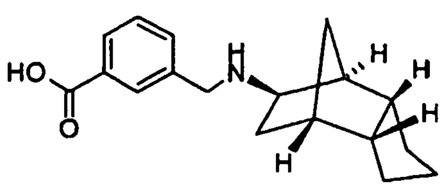

a) 3-[(Octahydro-4,7-methano-inden-5-ylamino)-methyl]-benzoesäure-butylester

1 g (3-Brom-benzyl)-(octahydro-4,7-methano-inden-5-yl)-amin-Hydrochlorid aus Beispiel 13), 115 mg 1,3-Bis (diphenylphosphino)propan, 63 mg Pd(II)acetat sowie 4 ml Tri-n-Butylamin wurden in 10 ml 1-Butanol sowie 2 ml DMF gelöst und unter CO-Atmosphäre (Normaldruck) 8 Stunden bei 110˚C gerührt. Anschließend wurden erneut 115 mg 1,3-Bis(diphenylphosphino)propan und 63 mg Pd(II)acetat zugegeben und weitere 7 Stunden bei 110˚C gerührt. Nach dem Abkühlen wurden 100 ml einer gesättigten wäßrigen $Na_2CO_3$-Lösung zugegeben und dreimal mit je 100 ml EE extrahiert. Über $MgSO_4$ wurde getrocknet und das Solvens im Vakuum entfernt. Chromatographie an Kieselgel mit DIP/ 2% Triethylamin lieferte 600 mg eines farblosen Öls.
$R_f$(DIP/2% Triethylamin) = 0,42; MS (ES+) : 342 $(M+H)^+$

b) 3-[(Octahydro-4,7-methano-inden-5-ylamino)-methyl]-benzoesäure

600 mg 3-[(Octahydro-4,7-methano-inden-5-ylamino)-methyl]-benzoesäure-butylester wurden in 1 ml n-Butanol gelöst und 2,1 ml einer 1 N wässrigen NaOH-Lösung zugegeben. 18 Stunden wurde bei RT, anschließend 4 Stunden bei 60 ˚C gerührt. Anschließend wurden die flüchtigen Bestandteile im Vakuum entfernt und zweimal mit je 5 ml Wasser n-Butanol-Reste unter vermindertem Druck azeotrop abdestilliert. Der Rückstand wurde mit 5 ml einer 10 %igen wässerigen HCl-Lösung aufgenommen, die flüchtigen Bestandteile im Vakuum entfernt und zweimal mit je 5 ml Toluol das Wasser unter vermindertem Druck azeotrop abdestilliert. Das Produkt enthielt noch beträchtliche Mengen Startmaterial, daher wurde es erneut in 6 ml Methanol gelöst und mit 1 ml einer 2N wäßrigen NaOH-Lösung versetzt. 3 Stunden wurde bei RT gerührt und anschließend weitere 5 ml einer 2N wässerigen NaOH-Lösung zugesetzt und 4 Stunden unter Rückfluß gekocht. Die flüchtigen Bestandteile wurden im Vakuum entfernt, mit 20 ml Wasser aufgenommen und mit verdünnter wäßriger HCl-Lösung auf pH = 7 gestellt. 1 Stunde wurde bei RT nachgerührt, das Produkt abgesaugt und im Vakuum getrocknet. Man erhielt 260 mg eines kristallinen Feststoffs, mp 258 - 261˚C.
MS (CI+) : 286 $(M+H)^+$

Beispiel 15: (exo/endo)-[3-(2-Methoxy-ethoxy)-benzyl]-(octahydro-4,7-methano-inden-5-yl)-amin-Hydrochlorid

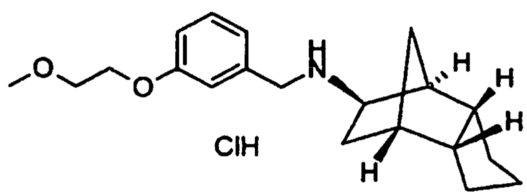

a) 3-(2-Methoxy-ethoxy)-benzaldehyd

1,0 g 3-Hydroxy-benzaldehyd, 1,1 g 1-Brom-2-methoxy-ethan und 10,7 g $Cs_2CO_3$ wurden in 10 ml DMF (wasserfrei) 4 Stunden bei 40˚C gerührt. Mit 100 ml Wasser wurde verdünnt und zweimal mit je 50 ml EE extrahiert. Über $MgSO_4$ wurde getrocknet und das Solvens im Vakuum entfernt. Man erhielt 1,3 g eines farblosen Öls.
$R_f$(DIP) = 0,24; MS (CI+) : 181 $(M+H)^+$

b) [3-(2-Methoxy-ethoxy)-benzyl]-(octahydro-4,7-methano-inden-5-yl)-amin-Hydrochlorid

300 mg 3-(2-Methoxy-ethoxy)-benzaldehyd, 253 mg (exo/endo)-Octahydro-4,7-methano-inden-5-ylamin (Amin 1) und 10 mg p-Toluolsulfonsäure wurden in 30 ml wasserfreiem Toluol gelöst und 5 Stunden unter Rückfluß gekocht. Die flüchtigen Bestandteile wurden im Vakuum entfernt und der Rückstand in 20 ml Methanol gelöst. 90 mg $NaBH_4$ wurden zugegeben und 2 Stunden bei RT gerührt. 18 Stunden wurde bei RT stehen gelassen, anschließend die flüchtigen Bestandteile im Vakuum entfernt. Mit 50 ml einer gesättigten wäßrigen $NaHCO_3$-Lösung wurde aufgenommen und dreimal mit je 50 ml EE extrahiert. Über $MgSO_4$ wurde getrocknet und das Solvens im Vakuum entfernt. Der Rückstand wurde mit 2 ml einer 10%igen wäßrigen HCl-Lösung aufgenommen und die flüchtigen Bestandteile im Vakuum entfernt. Der Rückstand wurde mit 10 ml Diethylether verrührt und man erhielt 163 mg des kristallinen Hydrochlorids, mp 134˚C.
$R_f$(EE) = 0,30; MS (CI+) : 316 $(M+H)^+$

Beispiel 16: (exo/endo)-(3-Iod-benzyl)-(octahydro-4,7-methano-inden-5-yl)-amin-Hydrochlorid

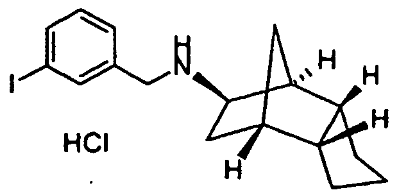

a) 1-Brommethyl-3-iod-benzol

4,4 g 3-Iod-tololuol wurden in 10 ml Chlorbenzol gelöst und bei 132°C portionsweise mit einem Gemisch aus 3,6 g N-Bromsuccinimid und 100 mg Dibenzoylperoxid versetzt. Eine Stunde wurde bei 132°C nachgerührt, nach dem Abkühlen mit 100 ml EE verdünnt und zunächst mit 100 ml einer gesättigten wäßrigen $Na_2SO_3$-Lösung und dann mit 100 ml einer gesättigten wäßrigen $Na_2CO_3$-Lösung gewaschen. Über $MgSO_4$ wurde getrocknet und das Solvens im Vakuum entfernt. Man erhielt 5,3 g eines blaßgelben Öls.
$R_f$(EE/HEP 1:8) = 0,44; MS (ES+) : 298 $(M+H)^+$

b) (3-Iod-benzyl)-(octahydro-4,7-methano-inden-5-yl)-amin-Hydrochlorid

755 mg (exo/endo)-Octahydro-4,7-methano-inden-5-ylamin (Amin 1) und 830 µl Triethylamin wurden in 20 ml wasserfreiem THF gelöst und bei 0°C langsam mit 2,8 g 1-Brommethyl-3-iod-benzol versetzt. 30 Minuten wurde bei 0°C, anschließend 5 Tage bei RT gerührt. Dann wurden 100 ml einer gesättigten wäßrigen $Na_2CO_3$-Lösung zugegeben und zweimal mit je 100 ml EE extrahiert. Über $MgSO_4$ wurde getrocknet und das Solvens im Vakuum entfernt. Der Rückstand wurde in 20 ml Methanol gelöst und mit einer 10%igen wäßrigen HCl-Lösung auf pH < 2 gestellt. Die flüchtigen Bestanteile wurden anschließend im Vakuum entfernt, der Rückstand mit 10 ml EE verrührt und im Vakuum getrocknet. Man erhielt 1,74 g farbloser Kristalle, mp 220 - 224°C (unter Zersetzung)
MS (Cl+) : 368 $(M+H)^+$

Beispiel 17: (exo/endo)-3-[(Octahydro-4,7-methano-inden-5-ylamino)-methyl]-benzonitril-Hydrochlorid

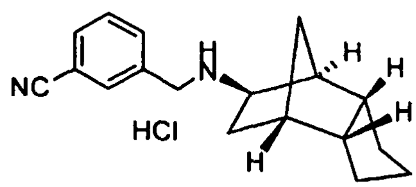

750 mg 3-Cyanobenzaldehyd, 865 mg (exo/endo)-Octahydro-4,7-methano-inden-5-ylamin (Amin 1) und 1,74 g Triethylamin wurden unter Feuchtigkeitsausschluß in 30 ml trockenem $CH_2Cl_2$ vorgelegt. Über ein Septum wurden 2,86 ml einer einmolaren Lösung von Titantetrachlorid in Toluol unter Rühren zugetropft. Nach 18 Stunden bei Raumtemperatur wurden 17,2 ml einer einmolaren Lösung von Natriumcyanoborhydrid in THF zugegeben und weitere 15 min gerührt. Anschließend wurden 20 ml 5 N Natriumhydroxid-Lösung und 60 ml Wasser zugesetzt, dann wurde dreimal mit 50 ml Ethylacetat extrahiert, getrocknet, filtriert und im Vakuum eingeengt. Der Rückstand wurde über Kieselgel filtriert ($CH_2Cl_2$/Methanol 97 : 3), erneut zur Trockne gebracht und das Rohprodukt mittels präparativer HPLC an RP-18 mit Acetonitril/Wasser (0,05% Trifluoressigsäure) gereinigt. Nach Gefriertrocknung werden 1,1 g des gewünschten Produkts als weißes Pulver in Form des Trifluoracetats erhalten.
250 mg dieses Pulvers wurden, wie in Beispiel 9) beschrieben, in das Hydrochlorid überführt. Es wurden 175 mg eines weißen Feststoffs erhalten.
MS (Cl+): 267,3 $(M+H)^+$

Beispiel 18: (exo/endo)-3-[(Octahydro-4,7-methano-inden-5-ylamino)-methyl]-benzoesäuremethyl ester-Hydrochlorid und

(exo/endo)-3-[(Octahydro-4,7-methano-inden-5-ylamino)-methyl)-benzoesäureethyl ester-Hydrochlorid

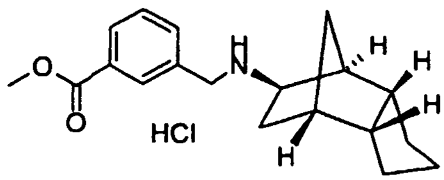

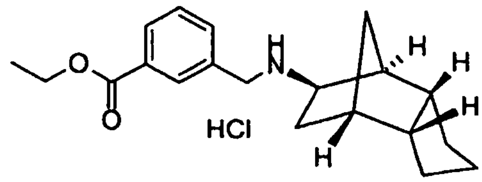

a) (exo/endo)-3-[(Octahydro-4,7-methano-inden-5-ylamino)-methyl]-benzimidinsäure-ethylester-Dihydrochlorid

500 mg 3-[(Octahydro-4,7-methano-inden-5-ylamino)-methyl]-benzonitril-Trifluoracetat aus Beispiel 17) wurden in 20 ml trockenem Ethanol (mit 5% Methanol und 5 Isopropanol vergällt) gelöst und unter Rühren und Eiskühlung 3 Stunden Chlorwasserstoff-Gas durch die Lösung geleitet. Über Nacht wurde bei Raumtemperatur stehengelassen und am nächsten Tag mit Stickstoff das überschüssige Chlorwasserstoff-Gas ausgetrieben und der Rückstand eingeengt. Es wurden 587 mg Benzamidinsäure-ethylester als weißes Pulver erhalten, das durch geringe Anteile Benzamidinsäure-methylester verunreinigt war.
Das Rohprodukt wurde direkt weiter umgesetzt.

b) (exo/endo)-3-[(Octahydro-4,7-methano-inden-5-ylamino)-methyl]-benzoesäure methyl ester-Hydrochlorid und

(exo/endo)-3-[(Octahydro-4,7-methano-inden-5-ylamino)-methyl]-benzoesäureethyl ester-Hydrochlorid

100 mg des Produktes aus a) wurden in 6 ml eines Gemisches aus Wasser und Trifluoressigsäure (5 : 1) gelöst und 3 Stunden bei Raumtemperatur gerührt. Nach Stehenlassen über Nacht wurde das Lösungsmittel entfernt und der Rückstand mittels präparativer HPLC an RP-18 mit Acetonitril/Wasser (0,05% Trifluoressigsäure) gereinigt. Die dabei anfallenden Trifluoracetat-Verbindungen des Ethyl- und Methylesters wurden mit Kaliumcarbonat-Lösung aufgenommen und mit Essigester extrahiert. Nach Trocknen und Abziehen des Essigesters wurde der Rückstand mit 2 N Salzsäure versetzt und gefriergetrocknet.
Es wurden 28 mg des Ethylesters und 7 mg des Methylesters erhalten.

Methylester: MS (ES+): 300,2 $(M+H)^+$
Ethylester: MS (ES+): 314,3 $(M+H)^+$

Beispiel 19: (exo/endo)-{3-[(Octahydro-4,7-methano-inden-5-ylamino)-methyl]-phenyl}-methanol-Hydrochlorid

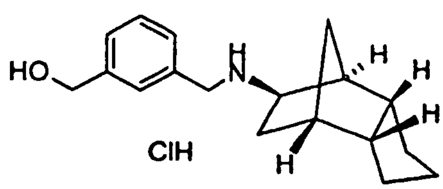

Zu 0,43 ml einer einmolaren THF-Lösung von Lithiumaluminiumhydrid wurden unter Rühren und Feuchtigkeitsausschluß 50 mg eines in 5 ml THF gelösten Methyl-/Ethylester-Gemisches, hergestellt wie in Beispiel 18 b) beschrieben, zugetropft. Nach Rühren bei Raumtemperatur und Stehenlassen über das Wochenende wurde unter Eiskühlung langsam Wasser zugetropft, der gebildete Niederschlag abgesaugt und gut mit Essigester gewaschen. Die wäßrige Phase wurde mit Essigester extrahiert und die vereinigten organischen Phasen wurden mit Magnesiumsulfat

getrocknet. Nach Abfiltrieren des Trockenmittels wurde das Lösungsmittel abgezogen und der Rückstand mittels präparativer HPLC an RP-18 mit Acetonitril/Wasser (0,05% Trifluoressigsäure) gereinigt. Anschließend wurde, wie in Beispiel 10) beschrieben, in das Hydrochlorid überführt.
Nach Gefriertrocknung wurden 7 mg Produkt erhalten.
MS (ES+): 272,2 $(M+H)^+$

Beispiel 20: (exo/endo)-3-[(Octahydro-4,7-methano-inden-5-ylamino)-methyl]-benzamid- Trifluoracetat

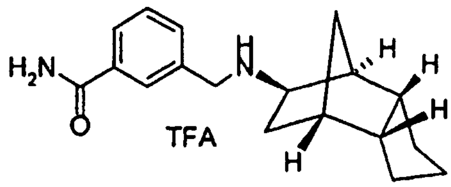

45 mg 3-[(Octahydro-4,7-methano-inden-5-ylamino)-methyl]-benzimidinsäure-ethylester-Dihydrochlorid aus Beispiel 18 a) wurden 8 Stunden auf 60°C erwärmt und anschließend drei Wochen bei Raumtemperatur stehengelassen. Der Feststoff wurde anschließend mittels präparativer HPLC an RP-18 mit Acetonitril/Wasser (0,05% Trifluoressigsäure) gereinigt. Nach Gefriertrocknung wurden 4 mg des gewünschten Produkts isoliert.
MS (ES+): 285,2 $(M+H)^+$

Beispiel 21: (exo/endo)-(3-Aminomethyl-benzyl)-(octahydro-4,7-methano-inden-5-yl)-amin-Bis-trifluoacetat

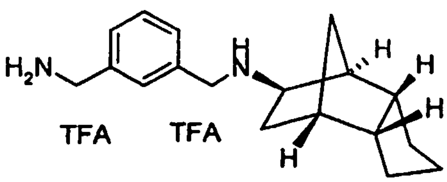

Zu 5 ml einer einmolaren Lösung von Lithiumaluminiumhydrid in THF wurden 100 mg 3-[(Octahydro-4,7-methano-inden-5-ylamino)-methyl]-benzonitril- Trifluoracetat aus Beispiel 17), gelöst in 5 ml trockenem THF, zugetropft. Anschließend wurde 5 Stunden auf 80°C erwärmt. Unter Eiskühlung wurde dann langsam Wasser zugetropft und mit Natronlauge versetzt. Der Niederschlag wurde abfiltriert und mit Ether gewaschen. Nach Extrahieren der wäßrigen Phase wurden die vereinigten organischen Phasen getrocknet und anschließend das Trockenmittel abfiltriert. Nach Einengen wurde der Rückstand mittels präparativer HPLC an RP-18 mit Acetonitril/Wasser (0,05% Trifluoressigsäure) gereinigt. Nach Gefriertrocknung wurden 26 mg Produkt isoliert.
MS (ES+): 271,2 $(M+H)^+$

Beispiel 22: (exo/endo)-3-[(Octahydro-4,7-methano-inden-5-ylamino)-methyl]-benzamidin-Bis-trifluoracetat

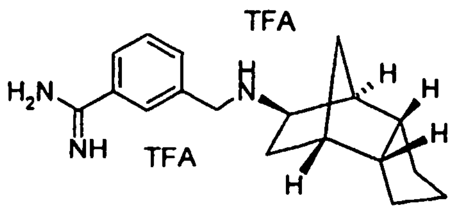

200 mg 3-[(Octahydro-4,7-methano-inden-5-ylamino)-methyl]-benzimidinsäure-ethylester- Dihydrochlorid aus Beispiel 18 a) wurden in 15 ml trockenem Ethanol gelöst und langsam ca. 20 ml Ammoniak einkondensiert. Nach 3 Stunden unter Rückfluss-Kochen in Ammoniak läßt man das Ammoniak über Nacht verdampfen. Der Rückstand wird eingeengt und dann mittels präparativer HPLC an RP-18 mit Acetonitril/Wasser (0,05% Trifluoressigsäure) gereinigt. Nach Gefriertrocknung werden 89 mg des gewünschten Produkts erhalten.
MS (Cl+): 284,3 $(M+H)^+$

Beispiel 23: (exo/endo)-(3-Nitro-benzyl)-(octahydro-4,7-methano-inden-5-yl)-amin-Hydrochlorid

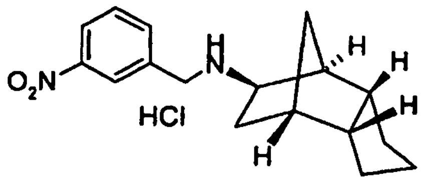

750 mg 3-Nitrobenzaldehyd, 751 mg (exo/endo)-Octahydro-4,7-methano-inden-5-ylamin (Amin 1) und 1,5 g Triethylamin wurden unter Feuchtigkeitsausschluß in 30 ml trockenem $CH_2Cl_2$ vorgelegt. Über ein Septum wurden 2,48 ml einer einmolaren Lösung von Titantetrachlorid in Toluol unter Rühren zugetropft. Nach 18 Stunden bei Raumtemperatur wurden 14,89 ml einer einmolaren Lösung von Natriumcyanoborhydrid in THF zugegeben und weitere 15 min. gerührt. Anschließend wurden 20 ml 5 N Natriumhydroxid-Lösung und 60 ml Wasser zugesetzt, dann wurde dreimal mit 50 ml Ethylacetat extrahiert, getrocknet, filtriert und im Vakuum eingeengt. Der Rückstand wurde über Kieselgel filtriert ($CH_2Cl_2$/Methanol 95:5), erneut zur Trockne gebracht und das Rohprodukt mittels präparativer HPLC an RP-18 mit Acetonitril/Wasser (0,05% Trifluoressigsäure) gereinigt. Ein Teil des so erhaltenen (3-Nitro-benzyl)-(octahydro-4,7-methano-inden-5-yl)-amin- Trifluoracetats wurde zwischen Essigester und Kaliumcarbonat-Lösung verteilt (pH 11). Die wäßrige Phase wurde noch dreimal mit Ethylacetat extrahiert und die vereinigten organischen Phasen getrocknet und eingeengt. Der Rückstand wurde mit 2 N Salzsäure und etwas Acetonitril aufgenommen und gefriergetrocknet. Es wurden 300 mg eines weißen Feststoffs erhalten.
MS (ES+): 287,2 $(M+H)^+$

Beispiel 24: (exo/endo)-(3-Amino-benzyl)-(octahydro-4,7-methano-inden-5-yl)-amin-Bis-trifluoracetat

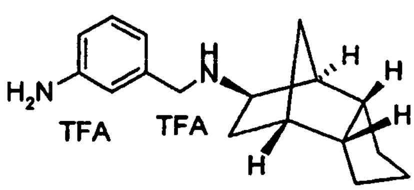

100 mg (3-Nitro-benzyl)-(octahydro-4,7-methano-inden-5-yl)-amin- Trifluoracetat aus Beispiel 23) wurden in einem Gemisch aus je 5 ml Ethanol und Eisessig gelöst. Anschließend wurden 57 mg Zinkpulver zugegeben und 4 Stunden bei 60°C gerührt. Es wurden dann weitere 25 g Zinkpulver zugegeben und nochmals zwei Stunden bei 60°C gerührt. Das Reaktionsgemisch wurde eingeengt, der Rückstand in Essigester aufgenommen und die organische Phase dreimal mit Kaliumcarbonat-Lösung gewaschen, getrocknet, filtriert und eingeengt. Der Rückstand wurde mittels präparativer HPLC an RP-18 mit Acetonitril/Wasser (0,05% Trifluoressigsäure) gereinigt. Nach Gefriertrocknung wurden 23 mg des gewünschten Produkts erhalten.
MS (ES+): 257,2 $(M+H)^+$

Beispiel 25: (exo/endo)-(3-Methoxy-benzyl)-methyl-(octahydro-4,7-methano-inden-5-yl)-amin-Hydrochlorid

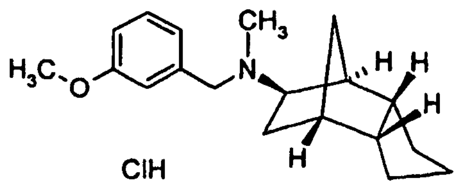

50 mg (exo/endo)-(3-Methoxy-benzyl)-(octahydro-4,7-methano-inden-5-yl)-amin aus Beispiel 5) wurden in 5 ml trockenem Aceton vorgelegt, 20 mg Kaliumcarbonat zugegeben, 30 min. gerührt und dann 9 µl Methyliodid zugetropft. Nach Stehenlassen übers Wochenende wurde das Reaktionsgemisch eingeengt, der Rückstand mit Wasser und Ethylacetat aufgenommen, die Phasen getrennt, die wäßrige Phase zweimal mit Ethylacetat extrahiert und die vereinigten organischen Phasen getrocknet, filtriert und eingeengt. Der Rückstand wird an Kieselgel mit Essigester/Heptan chromatographiert. Das erhaltene Amin wurde mit 2 N Salzsäure aufgenommen und gefriergetrocknet. Es werden 14 mg des gewünschten Produkts erhalten.
MS (Cl+): 286,4 $(M+H)^+$

Beispiel 26: (exo/endo)-(3-Methoxy-benzyl)-dimethyl-(octahydro-4,7-methano-inden-5-yl)-ammonium-Trifluoracetat

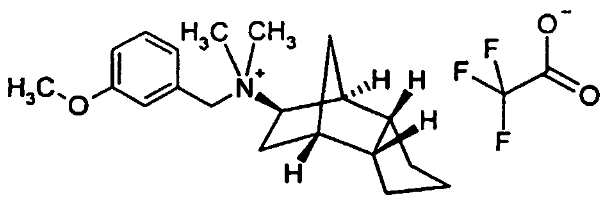

53 mg (exo/endo)-(3-Methoxy-benzyl)-(octahydro-4,7-methano-inden-5-yl)-amin aus Beispiel 5 wurden in 5 ml trockenem Aceton vorgelegt und dann 61 µl Methyliodid zugetropft. Nach Stehenlassen übers Wochenende wurden weitere 50 µl Methyljodid zugegeben. Nach Stehenlassen über Nacht wurden drei Tropfen N-Ethyldiisopropylamin zugegeben und weitere 5 Stunden gerührt. Dann wurde das Reaktionsgemisch eingeengt und mittels präparativer HPLC an RP-18 mit Acetonitril/Wasser (0,05% Trifluoressigsäure) gereinigt. Nach Gefriertrocknung wurden 53 mg des gewünschten Produkts erhalten.
MS (ES+): 300,3 $(M^+)$

Beispiel 27: (exo/exo)-(3-Fluoro-benzyl)-(octahydro-4,7-methano-inden-5-yl)-amin-Hydrochlorid

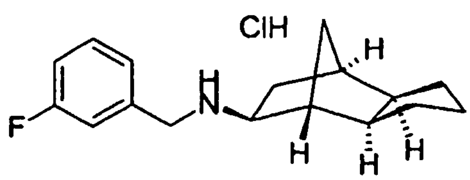

Eine Mischung aus 80 mg 3-Fluorbenzaldehyd, 97 mg (exo/exo)-Octahydro-4,7-methano-inden-5-ylamin (Amin 4), einer katalytischen Menge p-Toluolsulfonsäure und 7,5 ml wasserfreiem Toluol wurde 3 Stunden unter Rückfluß gekocht, das Toluol unter vermindertem Druck abdestilliert und der Rückstand in 5 ml Methanol gelöst. Zu dieser methanolischen Lösung fügte man unter Eiskühlung in kleinen Portionen 29 mg Natriumborhydrid und ließ auf Raumtemperatur kommen. Nach Zugabe von 2 N HCl filtrierte man den Niederschlag ab, löste ihn in heißem Ethanol und setzte in der Kälte Ether zu. Der so erhaltene Niederschlag wurde mit 2 N NaOH und Dichlormethan aufgenommen, die wäßrige Phase abgetrennt und die organische mit 2 N NaOH gewaschen. Anschließend wurde die organische Phase

mit $MgSO_4$ getrocknet, filtriert und eingeengt. Der Rückstand wurde dann mit 2 N HCl ins Hydrochlorid überführt. Es wurden 35 mg weißes Produkt erhalten.
MS (Cl+): 260,0 $(M+H)^+$

Beispiel 28: (exo/endo)-(2-Trifluormethyl-benzyl)-(octahydro-4,7-methano-inden-5-yl)-amin-Hydrochlorid

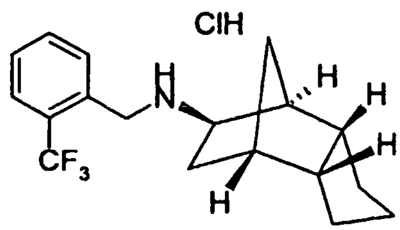

Zu einer Mischung aus 98 mg (exo/endo)-Octahydro-4,7-methano-inden-5-ylamin (Amin 1), 103 mg Diisopropylethylamin und 2 ml Dichlormethan werden unter Rühren langsam 158 mg 2-(Trifluormethyl)-benzylbromid, gelöst in 2 ml Dichlormethan, getropft. Nach Stehen über Nacht wird das Lösungsmittel abgezogen und der Rückstand mittels präparativer HPLC an RP-18 mit Acetonitril/Wasser (0,05% Trifluoressigsäure) gereinigt. Die Produkt-enthaltenden Fraktionen wurden vereinigt, das Acetonitril am Rotationsverdampfer entfernt, mit Kaliumcarbonat auf pH 11 gestellt und Ethylacetat zugegeben. Die wäßrige Phase wurde dreimal mit Ethylacetat extrahiert, die vereinigten Phasen getrocknet und eingeengt. Der Rückstand wurde mit 2 N Salzsäure und etwas Acetonitril aufgenommen und gefriergetrocknet. Nach Gefriertrocknung wurden 127 mg des gewünschten Produkts erhalten.
MS (Cl+): 310,2 $(M+H)^+$

Beispiel 29: (exo/endo)-(3-Dimethylamino-benzyl)-(octahydro-4,7-methano-inden-5-yl)-amin-Hydrochlorid

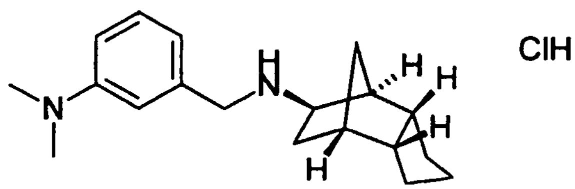

a) (exo/endo)-3-Dimethylamino-N-(octahydro-4,7-methano-inden-5-yl)-benzamid

Nach Zugabe von 1,78 g (0,011 Mol) N,N-Carbonyldiimidazol zu einer Lösung von 1,65 g (0,01 Mol) 3-N,N-Dimethylaminobenzoesäure in 40 ml wasserfreiem THF rührte man weitere 3 Stunden bei Raumtemperatur unter Argonatmosphäre und versetzte anschließend mit 1,82 g (0,012 Mol) (exo/endo)-Octahydro-4,7-methano-inden-5-ylamin (Amin 1). Man rührte eine Stunde bei Raumtemperatur und destillierte das Lösungsmittel nach dem Stehenlassen über Nacht ab. Der Rückstand wurde mit Wasser versetzt und mit 2 N HCl auf pH 3-4 gestellt. Nach magnetischer Rührung für ca. 30 Minuten filtrierte man das farblose kristalline (exo/endo)-3-Dimethylamino-N-(octahydro-4,7-methano-inden-5-yl)-benzamid ab, wusch den Feststoff mit Wasser und trocknete im Luftstrom. mp.: 152 - 156°C;
MS (Cl+): 299,4 $(M+H)^+$

b) (exo/endo)-(3-Dimethylamino-benzyl)-(octahydro-4,7-methano-inden-5-yl)-amin-Hydrochlorid

Zu einer Lösung aus 2 g (0,0067 Mol) (exo/endo)-3-Dimethylamino-N-(octahydro-4,7-methano-inden-5-yl)-benzamid in 100 ml wasserfreiem 1,2-Dimethoxyethan gab man zuerst 1,38 g (0,0097 Mol) Bortrifluorid Etherat und anschließend bei 10 - 15°C portionsweise 1,13 g (0,03 Mol) Natriumboranat. Nach mehrstündigem Erwärmen auf 70° und später auf 90°C und nach dem Stehenlassen über Nacht wurde das Lösungsmittel abdestilliert, der Rückstand mit

Wasser versetzt und mit 2N NaOH stark alkalisch gestellt. Man extrahierte 4-malig mit Ethylacetat, wusch die organische Phase mit Wasser, trocknete sie und verdampfte das Lösungsmittel. Nach der anschließenden Säulenchromatographie an Kieselgel mit einem Gemisch aus 1 Teil Ethylacetat und 3 Teilen Toluol versetzte man mit einer überschüssigen Lösung aus Chlorwasserstoff. Der Niederschlag (exo/endo)-(3-Dimethylamino-benzyl)-(octahydro-4,7-methano-inden-5-yl)-amin-Hydrochlorid wurde abfiltriert und getrocknet. Farbloser kristalliner Feststoff, mp.: 166 - 170˚C (unter Zersetzung).
MS (Cl+): 285,2 $(M+H)^+$

Beispiel 30: (exo/endo)-[2-(3-Methoxy-phenyl)-ethyl]-(octahydro-4,7-methano-inden-5-yl)-amin-Hydrochlorid

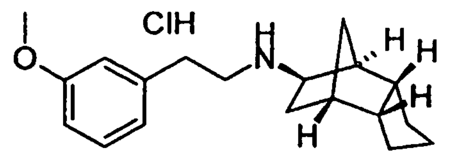

a) (exo/endo)-2-(3-Methoxy-phenyl)-N-(octahydro-4,7-methano-inden-5-yl)-acetamid

(exo/endo)-2-(3-Methoxy-phenyl)-N-(octahydro-4,7-methano-inden-5-yl)-acetamid erhielt man analog der in Beispiel 29 a) angegebenen Vorschrift aus 3-Methoxyphenylessigsäure, N,N'-Carbonyldiimidazol und (exo/endo)-Octahydro-4,7-methano-inden-5-ylamin (Amin 1). Gelbes, viskoses Öl.
MS (Cl+): 300,4 $(M+H)^+$

b) (exo/endo)-[2-(3-Methoxy-phenyl)-ethyl]-(octahydro-4,7-methano-inden-5-yl)-amin-Hydrochlorid

(exo/endo)-[2-(3-Methoxy-phenyl)-ethyl]-(octahydro-4,7-methano-inden-5-yl)-amin-Hydrochlorid erhielt man analog der in Beispiel 29 b) angegebenen Vorschrift durch Reduktion von (exo/endo)-2-(3-Methoxy-phenyl)-N-(octahydro-4,7-methano-inden-5-yl)-acetamid. Farbloser kristalliner Feststoff, mp.: 222 - 225˚C;
MS (ES+): 286,3 $(M+H)^+$

Beispiel 31: (exo/endo)-[3-(3-Methoxy-phenyl)-propyl]-(octahydro-4,7-methano-inden-5-yl)-amin-Hydrochlorid

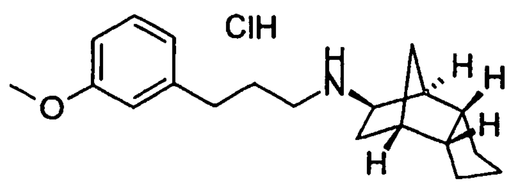

a) (exo/exo)-3-(3-Methoxy-phenyl)-N-(octahydro-4,7-methano-inden-5-yl)-propionamid

(exo/exo)-3-(3-Methoxy-phenyl)-N-(octahydro-4,7-methano-inden-5-yl)-propionamid erhielt man analog der in Beispiel 29 a) angegebenen Vorschrift aus 3-Methoxyphenylpropionsäure, N,N'-Carbonyldiimidazol und (exo/endo)-Octahydro-4,7-methano-inden-5-ylamin (Amin 1). Hellgelbes, öliges Produkt.
MS (Cl+): 314,0 $(M+H)^+$

b) (exo/endo)-[3-(3-Methoxy-phenyl)-propyl]-(octahydro-4,7-methano-inden-5-yl)-amin-Hydrochlorid

(exo/endo)-[3-(3-Methoxy-phenyl)-propyl]-(octahydro-4,7-methano-inden-5-yl)-amin-Hydrochlorid erhielt man analog der in Beispiel 29 b) angegebenen Vorschrift durch Reduktion von (exo/exo)-3-(3-Methoxy-phenyl)-N-(octahydro-4,7-methano-inden-5-yl)-propionamid. Farbloser kristalliner Feststoff, mp.: 186 - 188˚C.
MS (ES+): 300,3 $(M+H)^+$

Beispiel 32: (exo/endo)-(Decahydro-1,4-methano-naphthalin-2-yl)-(3-methoxybenzyl)-amin-Hydrochlorid

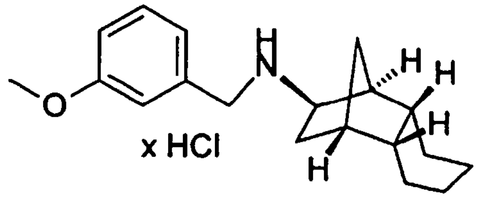

a) Bis-(3-chloro-1,2,3,4-tetrahydro-1,4-methano-naphthalin-2-yl)-diazen N,N'- dioxid

Zu einer Lösung von 3,56 g Benzonorbornadien [L.Friedman and F.M.Logullo, J.Org.Chem. 34: 3089 - 3092, (1969)] in 6 ml Eisessig und 6 ml Ethanol gab man 3,34 g iso-Amylnitrit und tropfte sodann 8,5 ml einer 15%igen Lösung von Chlorwasserstoffgas in Ethanol zu. Die entstehenden Suspension wurde weitere 2 ½ Stunden bei Raumtemperatur gerührt und anschließend mit 20 mg Diisopropylether versetzt. Nach weiterer Rührung über 30 Minuten filtrierte man den Feststoff ab. Heller kristalliner Feststoff; mp. 187 - 188˚C
MS (FAB): 415,1 $(M+H)^+$

b) (exo)-1,2,3,4-Tetrahydro-1,4-methano-naphthalin-2-ylamin

3 g Bis-(3-chloro-1,2,3,4-tetrahydro-1,4-methano-naphthalin-2-yl)-diazen N,N'- dioxid wurden in 150 ml Methanol suspendiert und mit Raney-Nickel Katalysator im Autoklaven mit Wasserstoff bei 100 bar, 100˚C über 20 Stunden hydriert. Nach dem Abfiltrieren des Katalysators verdampfte man das Lösungsmittel, versetzte den Rückstand mit Wasser, stellte mit NaOH stark alkalisch und extrahierte mehrfach mit Methyl-tert.butylether. Nach Trocknung der organischen Phasen erhielt man das gewünschte Amin als hellgelbe Flüssigkeit.
MS (ES+): 160,0 $(M+H)^+$

c) (exo/endo)-Decahydro-1,4-methano-naphhtalin-2-ylamin

Eine Lösung von 1 g exo-1,2,3,4-Tetrahydro-1,4-methano-naphthalin-2-ylamin in 10 ml Methanol und 30 ml 2 N Salzsäure wurde mit 0,4 g $RuO_2$ mit Wasserstoff bei 100 bar, 90˚C über 10 Stunden im Autoklaven hydriert. Nach Abtrennung des Katalysators wurde auf die Hälfte eingedampft, die so erhaltene wässrige Lösung mit 10 N NaOH stark alkalisch gestellt und mehrfach mit Methyl-tert.butylether extrahiert. Nach dem Trocknen und Verdampfen des Lösungsmittels erhielt man exo-Decahydro-1,4-methano-naphthalin-2-ylamin als farbloses Öl, das vorzugsweise unter Argon gelagert wurde.
MS (CI+): 166,2 $(M+H)^+$

d) (exo/endo)-(Decahydro-1,4-methano-naphthalin-2-yl)-(3-methoxy-benzyl)-amin-Hydrochlorid

0,97 g (exo/endo)-Decahydro-1,4-methano-naphthalin-2-ylamin wurden in 25 ml wasserfreiem Toluol gelöst und nach Zugabe von 0,8 g 3-Methoxybenzaldehyd und einer kleinen katalytischen Menge an p-Toluolsulfonsäure 3 Stunden am Rückflusskühler gekocht. Nach dem Verdampfen des Lösungsmittels löste man den Rückstand in 50 ml Methanol, gab portionsweise unter Rührung 0,26 g Natriumborhydrid zu und rührte die Mischung bei Raumtemperatur für ca. 20 Stunden. Man stellte die Lösung sodann mit einer Lösung aus Chlorwasserstoffgas in Methanol sauer, rührte 30 Minuten und filtrierte das präzipitierte Salz ab. Das Filtrat wurde eingedampft und der Rückstand aus einer Mischung aus Diisopropylether und Ethanol umkristallisiert. Farblose kristalline Substanz; mp. 234 - 236˚C
MS (ES+): 286,3 $(M+H)^+$

Die nachfolgend beschriebenen Verbindungen wurden entsprechend dem angegebenen Beispiel dargestellt:

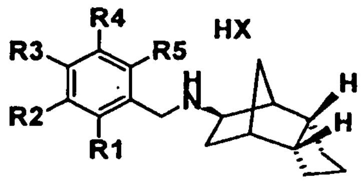

| Beispiel | analog Beispiel | R1 | R2 | R3 | R4 | R5 | HX | MS |
|---|---|---|---|---|---|---|---|---|
| 33 | 5 | -H | -H | $-OCH_3$ | -H | -H | HCl | CI+ $(M+H)^+$ 272,3 |
| 34 | 5 | $-OCH_3$ | -H | -H | -H | -H | HCl | CI+ $(M+H)^+$ 272,3 |
| 35 | 5 | -H | $-OCH_3$ | -H | -H | $OCH_3$ | HCl | ES+ $(M+H)^+$ 302,2 |
| 36 | 5 | -H | $-OCH_2O-$ | | -H | -H | HCl | ES+ $(M+H)^+$ 286,2 |
| 37 | 5 | -H | $-OCH_3$ | $-OCH_3$ | -H | -H | - | CI+ $(M+H)^+$ 302,4 |
| 38 | 5 | $-OCH_3$ | -H | $-OCH_3$ | -H | -H | HCl | ES+ $(M+H)^+$ 302,3 |
| 39 | 5 | -H | $-OCH_3$ | -F | -H | -H | HCl | CI+ $(M+H)^+$ 290,3 |
| 40 | 5 | -H | -OH | -H | -H | -H | HCl | CI+ $(M+H)^+$ 258,2 |
| 41 | 10 | -H | $-OCF_3$ | -H | -H | -H | TFA | ES+ $(M+H)^+$ 326,2 |
| 42 | 10 | -H | -OEt | -H | -H | -H | HCl | CI+ $(M+H)^+$ 286,3 |
| 43 | 10 | -H | $-OCF_2CF_2$ H | -H | -H | -H | TFA | ES+ $(M+H)^+$ 358,2 |
| 44 | 10 | -H | $-OPr^i$ | -H | -H | -H | HCl | CI+ $(M+H)^+$ 300,3 |
| 45 | 10 | -H | -OEt | $-OCH_3$ | -H | -H | TFA | ES+ $(M+H)^+$ 316,3 |

(fortgesetzt)

| Beispiel | analog Beispiel | R1 | R2 | R3 | R4 | R5 | HX | MS |
|---|---|---|---|---|---|---|---|---|
| 46 | 5 | -H | $-CH_3$ | -H | -H | -H | HCl | CI+ $(M+H)^+$ 256,3 |
| 47 | 10 | -H | -CF3 | -H | -H | -H | HCl | CI+ $(M+H)^+$ 310,3 |
| 48 | 5 | $OCH_3$ | $-CO_2CH_3$ | $-OCH_3$ | -H | -H | HCl | ES+ $(M+H)^+$ 360,2 |
| 49 | 11 | -H | -F | -F | -F | -H | HCl | CI+ $(M+H)^+$ 296, 3 |
| 50 | 5 | -H | -Cl | -H | -H | -H | HCl | CI+ $(M+H)^+$ 276,2 |
| 51 | 5 | -H | $-SO_2NH_2$ | -Cl | -H | -H | HCl | CI+ $(M+H)^+$ 355,1 |
| 52 | 5 | -H | 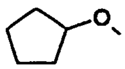 | -H | -H | -H | HCl | CI+ $(M+H)^+$ 326, 2 |
| 53 | 5 | -H | 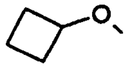 | -H | -H | -H | HCl | CI+ $(M+H)^+$ 312,2 |
| 54 | 5 | -H | 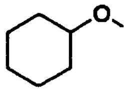 | -H | -H | -H | HCl | CI+ $(M+H)^+$ 340,2 |
| 55 | 28 | -H | -F | -F | -H | -H | HCl | ES+ $(M+H)^+$ 278,2 |
| 56 | 28 | -H | -OCH3 | -H | $-OCH_3$ | -H | HCl | ES+ $(M+H)^+$ 302,3 |
| 57 | 5 | -H | $-CH_2CH_3$ | -H | -H | -H | HCl | CI+ $(M+H)^+$ 270,1 |
| 58 | 28 | -F | -H | -H | -H | -H | HCl | CI+ $(M+H)^+$ 260,2 |
| 59 | 28 | $-SCF_3$ | -H | -H | -H | -H | HCl | CI+ $(M+H)^+$ 342,0 |

(fortgesetzt)

| | | | | | | | | |
|---|---|---|---|---|---|---|---|---|
| 60 | 28 | -H | -H | $-OCF_3$ | -H | -H | HCl | ES+ $(M+H)^+$ 326,2 |
| 61 | 5 | -H | -SCH3 | -H | -H | -H | HCl | ES+ $(M+H)^+$ 288,2 |
| 62 | 28 | -H | -H | $-CF_3$ | -H | -H | HCl | ES+ $(M+H)^+$ 310,2 |
| 63 | 9 | -OH | -OCH3 | -H | $-NO_2$ | -H | TFA | ES+ $(M+H)^+$ 333,2 |
| 64 | 9 | -H | 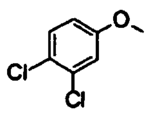 | -H | -H | -H | TFA | ES+ $(M+H)^+$ 402,2 ($^{35}Cl$) |

| Beispiel | | Analog Beispiel | MS |
|---|---|---|---|
| 65 | 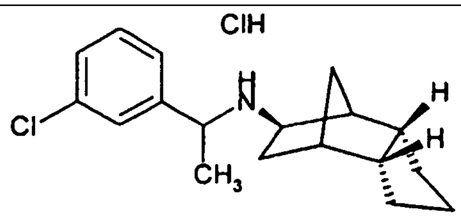 | 12 | ES+ $(M+H)^+$ 290,1 |
| 66 | 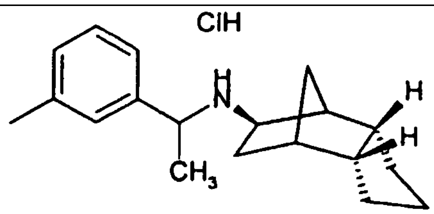 | 12 | ES+ $(M+H)^+$ 270,2 |
| 67 | 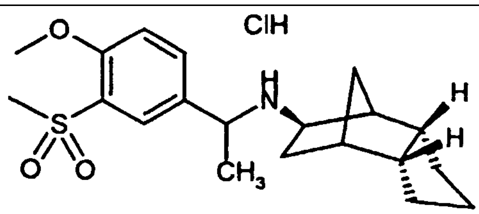 | 12 | ES+ $(M+H)^+$ 364,2 |
| 68 | 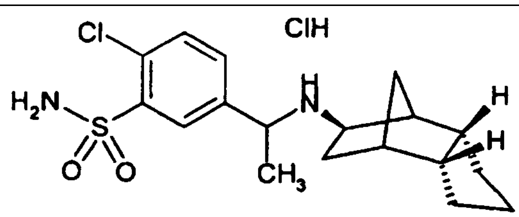 | 12 | ES+ $(M+H)^+$ 369,1 |

(fortgesetzt)

| Beispiel | | Analog Beispiel | MS |
|---|---|---|---|
| 69 | 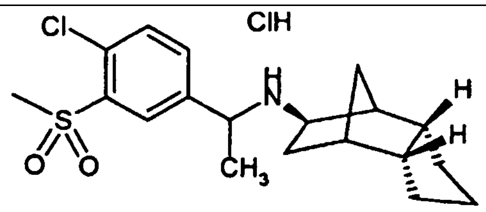 | 12 | ES+ (M+H) 368,2 |

Beispiel 70: (exo/endo)-(3-Methansulfonyl-benzyl)-(octahydro-4,7-methano-inden-5-yl)-amin-Hydrochlorid

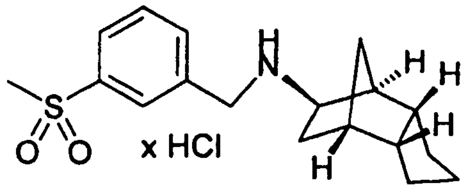

65 mg des Produkts aus Beispiel 61 wurden in 3 ml Methanol gelöst. Dann wurden 4 ml Natriumacetat-Puffer zugesetzt und das Gemisch auf 0˚C gekühlt. Nach langsamer Zugabe von 617 mg Oxone® wurde drei Stunden bei Raumtemperatur weitergerührt. Der Niederschlag wurde abfiltriert und das Filtrat im Vakuum konzentriert. Der Rückstand wurde mit Natriumhydrogencarbonat-Lösung versetzt und mit Ethylacetat extrahiert. Nach Trocknen und Filtrieren wurde im Vakuum konzentriert. Die erhaltenen 60 mg Rohprodukt wurden mittels präparativer HPLC über RP-18 mit Acetonitril/Wasser (0,05% Trifluoressigsäure) gereinigt. Die Produkt-enthaltenden Fraktionen wurden vereinigt, das Acetonitril am Rotationsverdampfer entfernt, mit Kaliumcarbonat auf pH 11 gestellt und Ethylacetat zugegeben. Die wäßrige Phase wurde dreimal mit Ethylacetat extrahiert, die vereinigten Phasen getrocknet und eingeengt. Der Rückstand wurde mit 2 N Salzsäure und etwas Acetonitril aufgenommen und gefriergetrocknet. Nach Gefriertrocknung wurden 8 mg des gewünschten Produkts erhalten.
MS (Cl+): 320,1 $(M+H)^+$

Pharmakologische Daten:

Beschreibung des Diurese- Versuchs:

Methode

Der Salidiurese-Versuch wurde an männlichen Wistar-Ratten im Gewicht von 155 bis 175 g durchgeführt. Den Tieren wurde 16 Stunden vor Versuchsbeginn das Futter, nicht jedoch das Trinkwasser, entzogen. Die Ratten wurden randomisiert in Diurese-Käfige gesetzt. Die Substanz von Beispiel 5) wurde in Trinkwasser gelöst und in einer Dosierung von 20 mg/kg Körpergewicht oral in einem Volumen von 10 ml/kg appliziert. Die Kontrollgruppe erhielt oral das entsprechende Volumen an Trinkwasser als Vehikel. Die Urinausscheidung jeder Gruppe wurde in den ersten 5 Stunden und in der Zeit von 6 - 24 Stunden gemessen. Die Harnelektrolyte Natrium und Kalium wurden flammenphotometrisch (Flammen-Photometer Eppendorf, Hamburg) und Chlorid potentiometrisch (Chloridmeter Eppendorf) bestimmt. Die Harnosmolalität wurde mit der Methode der Gefrierpunktserniedrigung (Osmometer Vogel, Gießen) ermittelt. Die Urin- und Elektrolytausscheidung sowie Osmolalität sind in ml/kg, mmol/kg bzw. mosmol/kg Körpergewicht angegeben. Das Verhältnis $Na^+/K^+$ läßt die Wirkqualität eines Diuretikums erkennen. Die Ergebnisse in der Tabelle repräsentieren arithmetische Mittelwerte mit Standardabweichung.

Ergebnisse:

| | | | | Harn | Na | K | Cl | Osmolalität | Na/K |
|---|---|---|---|---|---|---|---|---|---|
| | | | | ml/kg | mmol/kg | | | mosmol/kg | |
| | | | | | | | | | |
| Vehikel Kontrolle Trinkwasser Trinkwasser Trinkwasser 10 ml/kg KG p.o. n=5 | | Mittelwert | 1 -5- Stunden | 9,73 | 0,26 | 0,48 | 0,38 | 6,48 | 0,61 |
| | | SD | | 3,69 | 0,14 | 0,20 | 0,23 | 1,33 | 0,36 |
| | | | | | | | | | |
| | | Mittelwert | 6 - 24 Stunden | 26,84 | 1,75 | 3,95 | 1,44 | 32,32 | 0,45 |
| | | SD | | 6,44 | 0,47 | 0,93 | 0,40 | 7,17 | 0,12 |
| | | | | | | | | | |
| | | Mittelwert | Summe | 36,57 | 2,01 | 4,42 | 1,82 | 38,81 | 0,47 |
| | | SD | 1 - 24 Stunden | 9,08 | 0,37 | 0,97 | 0,26 | 7,00 | 0,11 |
| | | | | | | | | | |
| | | | | | | | | | |
| Beispiel 5 50 mg in 10 ml Trinkwasser /kg KG p.o. n=5 | | Mittelwert | 1 -5- Stunden | 12,39 | 0,31 | 0,75 | 0,60 | 7,82 | 0,47 |
| | | SD | | 8,03 | 0,27 | 0,43 | 0,37 | 3,08 | 0,31 |
| | | | | | | | | | |
| | | Mittelwert | 6 - 24 Stunden | 22,57 | 1,29 | 3,57 | 1,57 | 30,51 | 0,37 |
| | | SD | | 6,00 | 0,66 | 0,60 | 0,54 | 5,06 | 0,18 |
| | | | | | | | | | |
| | | Mittelwert | Summe | 34,96 | 1,60 | 4,31 | 2,17 | 38,33 | 0,38 |
| | | SD | 1 - 24 Stunden | 9,14 | 0,64 | 0,61 | 0,41 | 3,47 | 0,16 |

Beurteilung: Die Substanz von Beispiel 5) zeigte in einer Dosierung von 50 mg/kg oral an der Ratte keine salidiuretische Wirkung im Vergleich zur Kontrolle.

Beschreibung des Caco 2-Modells

Die Caco-2-Zellinie wurde bei American Type Culture Collection (ATCC) erworben und in Dulbecco's Modified Eagle Medium (hoher Glucoseanteil), ergänzt mit nichtessenziellen Aminosäuren, L-Glutamin, Penicillin/Streptomycin und 10 %igem fötalem Kälberserum, in einem Inkubator unter einer 10%igen $CO_2$-Atmosphäre bei 95%iger relativer Luftfeuchtigkeit und 37°C gehalten. Die Zellen wurden in Zellkulturkolben (175 $cm^2$) gezogen.
Für die Transport-Untersuchungen wurden die Caco-2-Zellen auf Polycarbonat Zellkultureinlagen (Costar Transwells® , Porengröße: 3 μm, Fläche: 4,71 $cm^2$) mit einer Zelldichte von 6,5 x $10^4$ Zellen/$cm^2$ ausgesät und in Sechs-Well-Kulturplatten mit Mediumwechsel nach vier und acht Tagen und dann jedem zweiten Tag inkubiert. Für die Experimente wurden 21 bis 25 Tage alte Monoschichten verwandt.

In jeder Testreihe wurde eine 21 Tage alte Monoschicht mit $^3$H-Dextran als Permeabilitätsmarker auf ihre Eigenschaften getestet. Der Wert der Transferrate (kumulativ) mußte nach 120 min im Bereich von 2% sein.

Nach Beseitigen des Wachstummediums von der apicalen und der basolateralen Seite wurden die Monoschichten mit dem Transport-Puffer (Hank's balanced salt solution pH 7,8; enthält 2,8 g/l Glucose) gespült, und die Zellen wurden 15 min bei 37°C unter 10%iger $CO_2$-Atmosphäre equilibriert. Danach wird der HBSS-Puffer wieder entfernt. Die Testverbindungen wurden in einem Gemisch aus HBSS-Puffer und DMSO gelöst und zum apicalen Puffer gegeben, so daß eine 1 %ige (V/V) DMSO-Lösung resultierte. Die Testkonzentration im ersten Versuch betrug 1 mM, im zweiten 100 μM. Die Versuche wurden bei 37°C durchgeführt und mit der Zugabe von 1,5 ml Testlösung auf der Donorseite (apical) gestartet. Transport-Puffer ohne Verbindung wurde auf die Empfängerseite (basolateral, 2,5 ml) gegeben. Zu verschiedenen Zeitpunkten wurden Proben von der basolateralen Seite genommen (1 ml) und durch frische 37°C warme Pufferlösung ersetzt. Apicale Proben wurden zu Beginn und am Ende (120 min) genommen, um anhand dieser Konzentrationen und der kumulativen basolateralen Konzentration die Wiederfindungsrate der Verbindungen zu bestimmen.

Die Verbindungen wurden mittels HPLC analysiert.
Der scheinbare Permeabilitätskoeffizient ($P_{app}$) wird über folgende Gleichung berechnet:

$$P_{app} = \frac{d_c \cdot V}{d_t \cdot A \cdot c_o}$$

darin bedeuten $d_c/d_t$ den Fluß durch die Monoschicht (μg oder Verbindung/ml x s), V das Flüssigkeitsvolumen in der Auffangkammer (ml), A die Oberflächengröße der Monoschicht ($cm^2$) und $c_0$ die Anfangskonzentration (μg oder Verbindung/ml) in der Donorkammer. Der Fluß durch die Monoschicht wurde aus der kumulativen basolateralen Konzentration zum entsprechende Zeitpunktpunkt unter Zuhilfenahme der anfänglich linearen Datenkurve (linear bis zu 60 min) errechnet. Die jeweiligen Bestimmungen wurden dreifach gemacht, so daß der berechnete $P_{app}$-Wert den Mittelwert dreier Messungen darstellt. $P_{app}$-Werte ausgewählter Verbindungen wurden mit literaturbekannten Absorptions-Werten korreliert und ergaben eine sigmoidale Kalibrierungskurve. Nach Untersuchungen von Artursson (Artursson P., Karlsson J.; Biochem. Biophys. Res. Comm. 1991;175/3: 880-885) läßt sich anhand dieser Kurve eine Aussage über den absorbierten Anteil einer Verbindung machen.

Ergebnisse:

| | | absorbierter Anteil [%] |
|---|---|---|
| Beispiel 5 | 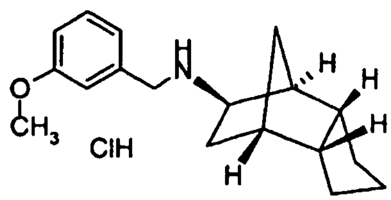 | 100 |
| Beispiel 10 | 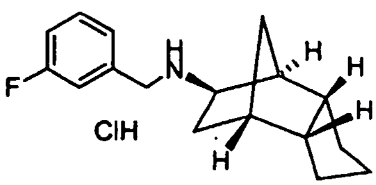 | 100 |
| S 3226 | 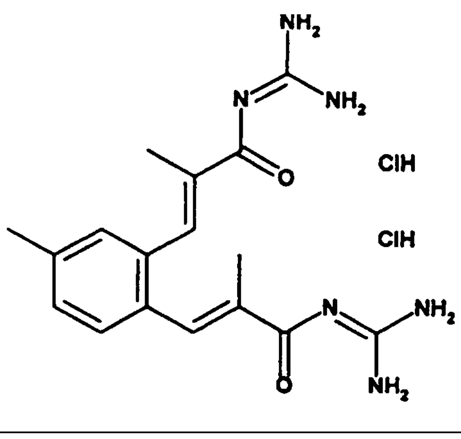 | <5 |

(fortgesetzt)

| | | absorbierter Anteil [%] |
|---|---|---|
| S 2120 | 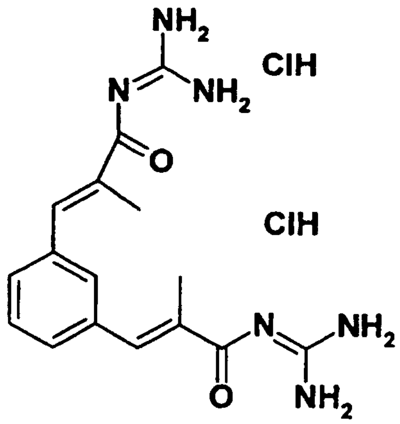 | <1 |

Gegenüber den literaturbekannten NHE3-aktiven Verbindungen vom AcylguanidinTyp (J.-R. Schwark et al. Eur. J. Physiol (1998) 436:797) zeigen die Verbindungen der Formel I oder I a eine deutlich überlegene Membrangängigkeit.

Beschreibung der NHE-Aktivitätsmessungen:

Die meisten der molekularbiologischen Techniken folgen Protokollen aus den Werken "Current Protocols in Molecular Biology (eds. Ausubel, F.M., Brent, R., Kingston, R.E., Moore, D.D., Seidman, J.G., Smith, J.A. und Struhl, K.; John Wiley & Sons)" bzw: "Molecular Cloning: A Laboratory Manual (Sambrock, J., Fritsch, E.F. und Maniatis, T.; Cold Spring Harbor Laboratory Press (1989))".

Im Rahmen unserer Arbeiten wurden stabil transfizierte Zellinien erzeugt, die jeweils einen der folgenden NHE-Subtypen exprimieren: NHE1 des Menschen (Sardet et al. (1989) Cell 56, 271-280), NHE2 des Kaninchens (Tse et al. (1993) J. Biol. Chem. 268, 11917 - 11924), NHE3 des Menschen (Brant et al. (1995) Am. J. Physiol. 269 (Cell Physiol. 38), C198 - C206) bzw. NHE3 der Ratte (Orlowski et al.; J. Biol. Chem. 267, 9331 - 9339 (1992)).

Die von Prof. Pouysségur erhaltenen cDNA-Klone der jeweiligen NHE-Subtypen wurden nach Anfügen geeigneter Linkersequenzen so in das Expressionsplasmid pMAMneo (erhältlich z. B. über CLONTECH, Heidelberg) einkloniert, daß die Erkennungssequenz für die Restriktionsendonuklease Nhel des Plasmids etwa 20 - 100 Basenpaare vor dem Startcodon des jeweiligen NHE-Subtyps liegt und die gesamte codierende Sequenz in dem Konstrukt vorhanden ist. Bei dem über RT-PCR aus humaner Nieren mRNA erhaltenem humanen NHE3 wurden die RT-PCR Primer so gewählt, daß die erhaltene cDNA-Bande an ihren Enden zu pMAMneo passende Schnittstellen aufweist.

Mit der sog. "Calciumphosphat-Methode" (beschrieben in Kapitel 9.1 von "Current Protocols in Molecular Biology") wurde die NHE-defiziente Zellinie LAP1 (Franchi et al.; Proc. Natl. Acad. Sci. USA 83, 9388 - 9392 (1986)) mit den Plasmiden, die die jeweiligen codierenden Sequenzen der NHE-Subtypen erhalten, transfiziert. Nach Selektion auf transfizierte Zellen über Wachstum in G418-haltigem Medium (nur Zellen, die durch Transfektion ein neo-Gen erhalten haben, können unter diesen Bedingungen überleben) wurde auf eine funktionelle NHE-Expression selektioniert. Dazu wurde die von Sardet beschriebene "Acid Load"-Technik verwendet (Sardet et al.; Cell 56, 271 - 280 (1989)). Zellen, die einen funktionsfähigen NHE-Subtypen exprimieren, können auch bei Abwesenheit von $CO_2$ und $HCO_3$- die bei diesem Test vorgenommene Ansäuerung kompensieren, untransfizierte LAP1-Zellen dagegen nicht. Nach mehrmaliger Wiederholung der "Acid Load" Selektion wurden die überlebenden Zellen in Mikrotiterplatten so ausgesät, daß statistisch eine Zelle pro Well vorkommen sollte. Unter dem Mikroskop wurde nach etwa 10 Tagen überprüft, wie viele Kolonien pro Well wuchsen. Zellpopulationen aus Einzelkolonien wurden dann mit dem XTT-Proliferation Kit (Boehringer Mannheim) hinsichtlich ihrer Überlebensfähigkeit nach "Acid Load" untersucht. Die besten Zellinien wurden für die weiteren Tests verwendet und zur Vermeidung eines Verlustes der transfizierten Sequenz unter-ständigem Selektionsdruck in G418-haltigem Medium kultiviert. Zur Bestimmung von $IC_{50}$-Werten für die Hemmung der einzelnen NHE-Subtypen durch spezifische Substanzen wurde ein von S. Faber entwickelter Test (Faber et al.; Cell. Physiol. Biochem. 6, 39 - 49 (1996)), der auf der "Acid Load" Technik beruht, leicht abgewandelt.

In diesem Test wurde die Erholung des intrazellulären pHs ($pH_i$) nach einer Ansäuerung ermittelt, die bei funktionsfähigem NHE auch unter bicarbonatfreien Bedingungen einsetzt. Dazu wurde der $pH_i$ mit dem pH-sensitiven Fluoreszenzfarbstoff BCECF (Calbiochem, eingesetzt wird die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den $pH_i$ umgerechnet. Abweichend von dem beschriebenen Protokoll

wurden die Zellen bereits bei der BCECF-Beladung in $NH_4Cl$-Puffer (pH 7,4) inkubiert ($NH_4Cl$-Puffer 115 mM NaCl, 20 mM $NH_4Cl$, 5 mM KCl, 1 mM $CaCl_2$, 1 mM $MgSO_4$, 20 mM Hepes, 5 mM Glucose, 1 mg/ml BSA; mit 1 M NaOH wird ein pH von 7,4 eingestellt). Die intrazelluläre Ansäuerung wurde durch Zugabe von 975 µl eines $NH_4Cl$-freien Puffers zu 25 µl Aliquots der in $NH_4Cl$-Puffer inkubierten Zellen induziert. Die nachfolgende Geschwindigkeit der pH-Erholung wurde bei NHE1 2 Minuten, bei NHE2 5 Minuten und bei NHE3 3 Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in $Na^+$-haltigem Puffer inkubiert (133,8 mM NaCl, 4,7 mM KCl, 1,25 mM $CaCl_2$, 1,25 mM $MgCl_2$, 0,97 mM $Na_2HPO_4$, 0,23 mM $NaH_2PO_4$, 5 mM Hepes, 5 mM Glucose, mit 1 M NaOH wird ein pH von 7,0 eingestellt). Für die Bestimmung des 0%-Wertes wurden die Zellen in einem $Na^+$-freien Puffer inkubiert (133,8 mM Cholinchlorid, 4,7 mM KCl, 1,25 mM $CaCl_2$, 1,25 mM $MgCl_2$, 0,97 mM $K_2HPO_4$, 0,23 mM $KH_2PO_4$, 5 mM Hepes, 5 mM Glucose, mit 1 M NaOH wird ein pH von 7,0 eingestellt). Die zu testenden Substanzen wurden in dem $Na^+$-haltigem Puffer angesetzt. Die Erholung des intrazellulären pHs bei jeder getesteten Konzentration einer Substanz wurde in Prozent der maximalen Erholung ausgedrückt. Aus den Prozentwerten der pH-Erholung wurde mittels des Programms SigmaPlot der $IC_{50}$-Wert der jeweiligen Substanz für die einzelnen NHE-Subtypen berechnet.

NHE3-Aktivität

| Beispiel | Ratten-NHE3 $IC_{50}$ [µM] |
|---|---|
| 5 | 0,81 |
| (+)-6 | 0,5 |
| (-)-6 | 1 |
| 10 | 0,9 |
| 9 | 5 |
| 8 | 70 |
| 7 | 31 |

**Patentansprüche**

1. Substituierte Norbornylamino-Derivate mit exo-konfiguriertem Stickstoff und endo-anelliertem Fünf-, Sechs- oder Siebenring der Formel I und mit exo-konfiguriertem Stickstoff und exo-anelliertem Fünf-, Sechs- oder Siebenring der Formel I a,

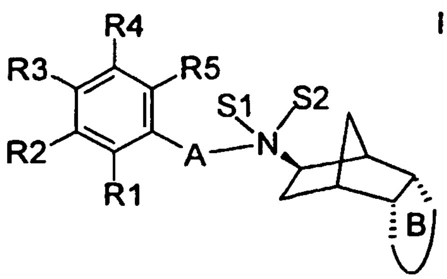
I

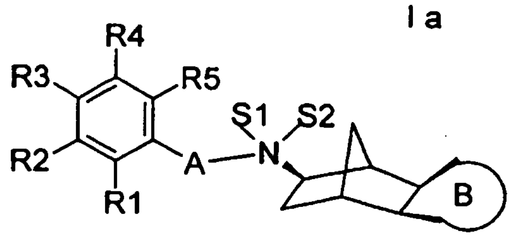
I a

worin bedeuten:

A ($C_1$-$C_4$)- Alkylen;
S1 ein freies Elektronenpaar oder ($C_1$-$C_4$)- Alkyl;
S2 ($C_1$-$C_4$)- Alkyl oder H;
wobei, wenn S1 und S2 Alkyl bedeuten, $X^-$ in der resultierenden Gruppierung [-$N^+$(S1S2)- $X^-$] einem pharmakologisch akzeptablem Anion oder Trifluoracetat entspricht;
B ein gesättigter oder ungesättigter Kohlenstoff-Fünf-, Sechs- oder Siebenring, der mit Oxo, Hydroxy, ($C_1$-$C_4$)-Alkoxy und ($C_1$-$C_4$)-Alkyl einfach oder mehrfach unabhängig voneinander substituiert sein kann;

und

R1, R2, R3, R4 und R5 unabhängig voneinander H, OH, F, Cl, Br, I, CN, $NO_2$, Amidino, $-CO_2R(11)$, -CONR(11)R(12), $-SO_rR(11)$,$-SO_sNR(11)R(12)$, ($C_1$-$C_4$)-Alkyl, ($C_1$-$C_4$)-Alkoxy, ($C_1$-$C_4$)-Alkoxy($C_1$-$C_4$)- alkyl, ($C_1$-$C_4$)-Alkoxy-($C_1$-$C_4$)-alkyloxy, Hydroxy-($C_1$-$C_4$)- alkyl, ($C_3$-$C_7$)- Cycloalkoxy oder Phenyloxy,
wobei Phenyl unsubstituiert ist oder substituiert mit bis zu drei Substituenten, die unabhängig voneinander sind, ausgewählt aus der Gruppe bestehend aus F, Cl, Br und Methoxy;
Amino, ($C_1$-$C_4$)- Alkylamino, Di-($C_1$-$C_4$)- alkylamino, Amino-($C_1$-$C_4$)- alkyl, Di-($C_1$-$C_4$)- alkylamino-($C_1$-$C_4$)- alkyl, ($C_1$-$C_4$)-Alkylamino-($C_1$-$C_4$)- alkyl, wobei die Wasserstoffatome in den Alkylresten ganz oder teilweise durch Fluor substituiert sein können;

R11 und R12 unabhängig voneinander H oder ($C_1$-$C_4$)- Alkyl,
wobei die Wasserstoffatome in den Alkylresten ganz oder teilweise durch Fluor substituiert sein können;
r 0, 1 oder 2;
s 1 oder 2;

oder

R1 und R2, R2 und R3, R3 und R4 oder R4 und R5 jeweils gemeinsam eine Gruppe $-O-(CH_2)_n-O-$;

n 1 oder 2;

und
die jeweils verbleibenden Reste R1, R2, R3, R4 oder R5
unabhängig voneinander H, OH, F, Cl, Br, I, CN, $NO_2$, Amidino, $-CO_2R(11)$, -CONR(11)R(12), $-SO_rR(11)$,$-SO_SNR(11)R(12)$, ($C_1$-$C_4$)-Alkyl, ($C_1$-$C_4$)-Alkoxy, ($C_1$-$C_4$)-Alkoxy-($C_1$-$C_4$)-alkyl, ($C_3$-$C_7$)- Cycloalkoxy, Hydroxy-($C_1$-$C_4$)-alkyl, Amino, ($C_1$-$C_4$)- Alkylamino, Di-($C_1$-$C_4$)- alkylamino, Amino-($C_1$-$C_4$)- alkyl, Di-($C_1$-$C_4$)-alkylamino-($C_1$-$C_4$)- alkyl, ($C_1$-$C_4$)-Alkylamino-($C_1$-$C_4$)- alkyl,
wobei die Wasserstoffatome in den Alkylresten ganz oder teilweise durch Fluor substituiert sein können;

R11 und R12 unabhängig voneinander H oder ($C_1$-$C_4$)- Alkyl,
wobei die Wasserstoffatome in den Alkylresten ganz oder teilweise durch Fluor substituiert sein können;
r 0, 1 oder 2;
s 1 oder 2;

wobei Benzyl-(octahydro-4,7-methano-inden-5-yl)-amin ausgenommen ist,
sowie deren pharmazeutisch verträgliche Salze oder Trifluoracetate.

**2.** Verbindungen der Formel I oder I a nach Anspruch 1 mit exo-konfiguriertem Stickstoff und endo-anelliertem Fünf- oder Sechsring der Formel I und mit exo-konfiguriertem Stickstoff und exo-anelliertem Fünf- oder Sechsring der Formel I a, in der bedeuten:

A ($C_1$-$C_2$)- Alkylen
S1 freies Elektronenpaar oder Methyl,
S2 H;
B ein gesättigter oder ungesättigter Kohlenstoff-Fünf- oder Sechsring;
R1, R2, R3, R4 und R5 unabhängig voneinander H, Amino, Hydroxymethyl, OH, Methoxy, F, Cl, Br oder Iod;

oder

R2 und R3 gemeinsam $-O-CH_2-O-$;

und
die verbleibenden Reste R1, R4 und R5
unabhängig voneinander H, OH, F, Cl, Br, I, CN, $NO_2$, ($C_1$-$C_2$)- Alkoxy, Amino, ($C_1$-$C_2$)-Alkylamino oder Di-($C_1$-$C_2$)-alkylamino,
wobei die Wasserstoffatome in den Alkylresten ganz oder teilweise durch Fluor substituiert sein können;
sowie deren pharmazeutisch verträgliche Salze oder Trifluoracetate.

**3.** Verbindungen der Formel I oder I a nach Anspruch 1 und 2 mit exo-konfiguriertem Stickstoff und endo-anelliertem Fünf- oder Sechsring der Formel I und mit exo-konfiguriertem Stickstoff und exo-anelliertem Fünf- oder Sechsring der Formel I a, in der bedeuten:

A ($C_1$-$C_2$)-Alkylen;
S1 freies Elektronenpaar;
S2 H;
B ein gesättigter oder ungesättigter Kohlenstoff-Fünf oder Sechsring;
R1, R3 und R5 Wasserstoff;

und R2 und R4
unabhängig voneinander H, Methoxy, F oder Cl;
oder

R2 und R3 gemeinsam -O-$CH_2$-O-;

und

R1, R4 und R5 Wasserstoff;

sowie deren pharmazeutisch verträgliche Salze.

**4.** Verbindungen der Formel I oder I a nach einem oder mehreren der Ansprüche 1 bis 3 mit exo-konfiguriertem Stickstoff und endo-anelliertem Fünf- oder Sechsring der Formel I und mit exo-konfiguriertem Stickstoff und exo-anelliertem Fünfring der Formel I a , **dadurch gekennzeichnet, dass** es sich um folgende Verbindungen handelt:

exo/endo-(3-Chlor-benzyl)-(octahydro-4,7-methano-inden-5-yl)-amin,
exo/endo-(3-Fluor-benzyl)-(octahydro-4,7-methano-inden-5-yl)-amin;
exo/endo-Benzo[1,3]dioxol-5-ylmethyl-(octahydro-4,7-methano-inden-5-yl)-amin,
exo/endo-(*rac*)-(3-Methoxy-benzyl)-(octahydro-4,7-methano-inden-5-yl)-amin,
exo/endo-(+)-(3-Methoxy-benzyl)-(octahydro-4,7-methano-inden-5-yl)-amin,
exo/endo-(-)-(3-Methoxy-benzyl)-(octahydro-4,7-methano-inden-5-yl)-amin,
exo/endo-[1-(3-Methoxy-phenyl)-ethyl]-(octahydro-4,7-methano-inden-5-yl)-amin,
exo/endo-(3-Fluor-benzyl)-(3a,4,5,6, 7,7a-hexahydro-1H-4,7-methano-inden-5-yl)-amin,
exo/endo-(3-Fluor benzyl)-(3a,4,5,6,7,7a-hexahydro-3H-4,7-methano-inden-5-yl)-amin,
exo/endo-(3a,4,5,6,7,7a-Hexahydro-1 H-4,7-methano-inden-5-yl)-(3-methoxybenzyl)-amin,
exo/endo-(3a,4,5,6,7,7a-Hexahydro-3H-4,7-methano-inden-5-yl)-(3-methoxybenzyl)-amin,
exo/endo-(Decahydro-1,4-methano-naphthalin-2-yl)-(3-methoxy-benzyl)-amin,
exo/endo-(3,5-Difluor-benzyl)-(octahydro-4,7-methano-inden-5-yl)-amin,
exo/exo-(3-Fluor-benzyl)-(octahydro-4,7-methano-inden-5-yl)-amin und
exo/exo-(3-Methoxy-benzyl)-(octahydro-4,7-methano-inden-5-yl)-amin
sowie deren pharmazeutisch akzeptable Salze oder Trifluoracetate.

**5.** Verbindungen der Formel I nach einem oder mehreren der Ansprüche 1 bis 4, mit exo-ständigem Amin und endo-anelliertem Fünf- oder Sechsring, welche sind:

exo/endo-(3-Chlor-benzyl)-(octahydro-4,7-methano-inden-5-yl)-amin,
exo/endo-(3-Fluor-benzyl)-(octahydro-4,7-methano-inden-5-yl)-amin
exo/endo-(3-Fluor-benzyl)-(3a,4,5,6,7,7a-hexahydro-1H-4,7-methano-inden-5-yl)-amin,
exo/endo-(3-Fluor-benzyl)-(3a,4,5,6,7,7a-hexahydro-3H-4,7-methano-inden-5-yl)-amin,
exo/endo-Benzo[1,3]dioxol-5-ylmethyl-(octahydro-4,7-methano-inden-5-yl)-amin,
exo/endo-(*rac*)-(3-Methoxy-benzyl)-(octahydro-4,7-methano-inden-5-yl)-amin,
exo/endo-(+)-(3-Methoxy-benzyl)-(octahydro-4,7-methano-inden-5-yl)-amin,
exo/endo-(Decahydro-1,4-methano-naphthalin-2-yl)-(3-methoxy-benzyl)-amin,
exo/endo-(-)-(3-Methoxy-benzyl)-(octahydro-4,7-methano-inden-5-yl)-amin und
exo/endo-(3,5-Difluor-benzyl)-(octahydro-4,7-methano-inden-5-yl)-amin,
sowie deren pharmazeutisch verträgliche Salze oder Trifluoracetate.

**6.** Verfahren zur Herstellung der Verbindungen der Formel I oder I a nach Anspruch 1, **dadurch gekennzeichnet, daß** man

a) eine Verbindung der Formel II oder II a

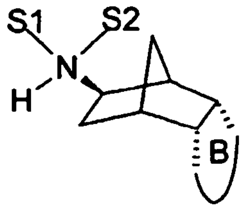

II

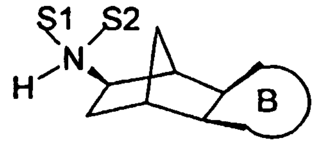

II a

mit einer Verbindung der Formel III in Gegenwart geeigneter Reduktionsmittel und möglicherweise auch Lewis-Säuren direkt zu Verbindungen der Formel I oder I a umsetzt,

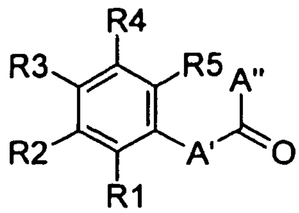

III

worin S1, S2, B, R1, R2, R3, R4 und R5 die oben angegebene Bedeutung besitzen, während unabhängig voneinander A' einer Bindung oder ($C_1$-$C_3$)-Alkyl und A" H oder ($C_1$-$C_3$)- Alkyl entspricht und A' und A" zusammen mit dem Kohlenstoffatom der Carbonylgruppe so viele Kohlenstoffatome repräsentieren wie A;
oder
b) das aus Verbindungen der Formeln II oder II a und III gebildete Intermediat der Formel IV oder IV a,

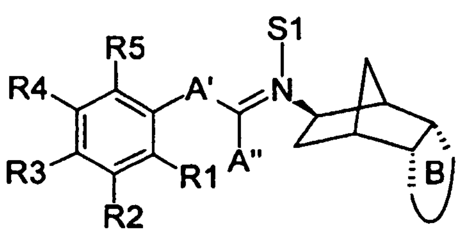

IV

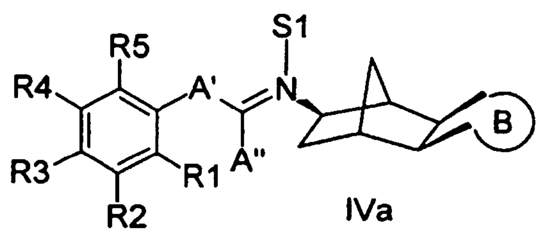

IVa

worin wenn S1 ($C_1$-$C_4$)-Alkyl entspricht, ein Onium-Stickstoff gebildet wird, dem ein Gegenion wie beispielsweise Chlorid oder Tosylat zugeordnet ist, isoliert und dann mit geeigneten Reduktionsmitteln in die Verbindungen der Formel I oder I a überführt,
oder
c) eine Verbindung der Formel II oder II a mit einem Alkylierungsmittel der Formel V,

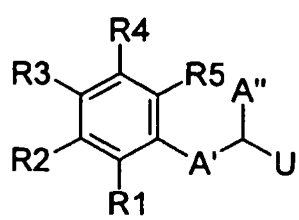

V

in der U für eine nukleophil substituierbare Gruppe steht - wie Chlor, Brom, und Iod sowie Mesylat, Tosylat oder Triflat- und die anderen Reste wie oben beschrieben definiert sind, aber hier dem Kohlenstoffatom der Carbonylgruppe das Kohlenstoffatom, an das U gebunden ist, entspricht, vorzugsweise in Gegenwart von nicht nukleophilen Basen wie Diisopropylethylamin umsetzt,
oder
d) Carbonsäureamide der Formel VI oder VI a,

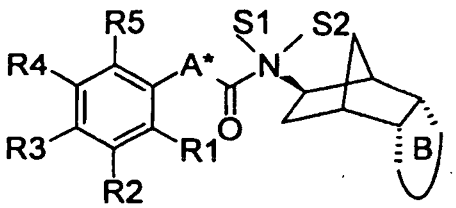
VI

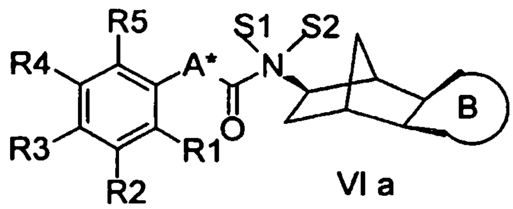
VI a

worin A* einer Bindung oder ($C_1$-$C_3$)-Alkyl entspricht und die anderen Reste, wie oben beschrieben, definiert sind,
zu den entsprechenden Aminen reduziert;
oder
e) Verbindungen der Formel I oder I a, in denen S1 einem freien Elektronenpaar und S2 Wasserstoff entspricht, mit Alkylierungsmitteln der Formel VII,

S*-U VII

worin S* ($C_1$-$C_4$)-Alkyl bedeutet und U oben beschriebene Bedeutung besitzt, mono- oder dialkyliert, so daß aus dieser Umsetzung tertiäre Amine oder quartäre Ammoniumsalze hervorgehen;
oder
f) einen Dicyclopentadienylplatin-Komplex der Formel VIII,

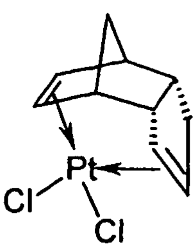
VIII

mit Aminen vom Typ der Formel IX,

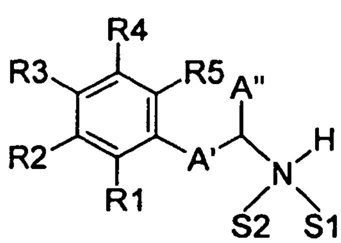
IX

worin S1, S2, R1, R2, R3, R4 und R5 die oben angegebene Bedeutung besitzen, während unabhängig voneinander A' einer Bindung oder ($C_1$-$C_3$)-Alkyl und A" H oder ($C_1$-$C_3$)-Alkyl entspricht und A' und A" zusammen mit dem Kohlenstoffatom, an welches das Stickstoffatom gebunden ist, so viele Kohlenstoffatome repräsentieren wie A,

umsetzt und anschließend das gebildete Zwischenprodukt zu Verbindungen der Formel I reduziert; und daß man gegebenenfalls in das pharmazeutisch verträgliche Salz oder Trifluoracetat überführt.

**7.** Verwendung einer Verbindung I oder I a nach Anspruch 1 zur Herstellung eines Medikaments zur Behandlung oder Prophylaxe von Störungen des Atemantriebs.

**8.** Verwendung einer Verbindung I oder I a nach Anspruch 1 zur Herstellung eines Medikaments zur Behandlung oder Prophylaxe von Atemstörungen, insbesondere Schlaf-bedingten Atemstörungen wie Schlafapnoen.

**9.** Verwendung einer Verbindung I oder I a nach Anspruch 1 zur Herstellung eines Medikaments zur Behandlung oder Prophylaxe des Schnarchens.

**10.** Verwendung einer Verbindung I oder I a nach Anspruch 1 zur Herstellung eines Medikaments zur Behandlung oder zur Prophylaxe von akuten und chronischen Nierenerkrankungen, besonders des akuten Nierenversagens und des chronischen Nierenversagens.

**11.** Verwendung einer Verbindung I oder I a nach Anspruch 1 zur Herstellung eines Medikaments zur Behandlung oder Prophylaxe von Störungen der Darmfunktion.

**12.** Verwendung einer Verbindung I oder I a nach Anspruch 1 zur Herstellung eines Medikaments zur Behandlung oder Prophylaxe von Störungen der Gallenfunktion.

**13.** Verwendung einer Verbindung I oder I a nach Anspruch 1 zur Herstellung eines Medikaments zur Behandlung oder Prophylaxe von ischämischen Zuständen des peripheren und zentralen Nervensystems und des Schlaganfalls.

**14.** Verwendung einer Verbindung I oder I a nach Anspruch 1 zur Herstellung eines Medikaments zur Behandlung oder Prophylaxe von ischämischen Zuständen peripherer Organe und Gliedmaßen.

**15.** Verwendung einer Verbindung I oder I a nach Anspruch 1 zur Herstellung eines Medikaments zur Behandlung von Schockzuständen.

**16.** Verwendung einer Verbindung I oder I a nach Anspruch 1 zur Herstellung eines Medikaments zu Einsatz bei chirurgischen Operationen und Organtransplantationen.

**17.** Verwendung einer Verbindung 1 oder I a nach Anspruch 1 zur Herstellung eines Medikaments zur Konservierung und Lagerung von Transplantaten für chirurgische Maßnahmen.

**18.** Verwendung einer Verbindung I oder I a nach Anspruch 1 zur Herstellung eines Medikaments zur Behandlung von Krankheiten, bei denen die Zellproliferation eine primäre oder sekundäre Ursache darstellt.

**19.** Verwendung einer Verbindung I oder I a nach Anspruch 1 zur Herstellung eines Medikaments zur Behandlung oder Prophylaxe von Störungen des Fettstoffwechsels.

**20.** Verwendung einer Verbindung I oder I a nach Anspruch 1 zur Herstellung eines Medikaments zur Behandlung oder Prophylaxe des Befalls durch Ektoparasiten.

**21.** Heilmittel, enthaltend eine wirksame Menge einer Verbindung I oder I a nach einem oder mehreren der Ansprüche 1 bis 5.

## Claims

**1.** A substituted norbornylamino derivative having exo-configured nitrogen and an endo-fused five-, six- or seven-membered ring of the formula I or having exo-configured nitrogen and an exo-fused five-, six- or seven-membered ring of the formula I a

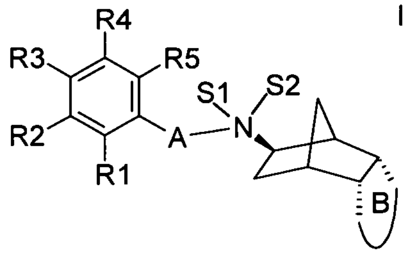

I

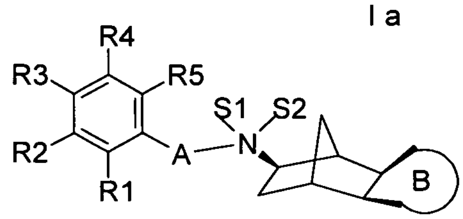

I a

in which:

A is ($C_1$-$C_4$)-alkylene;
S1 is a free electron pair or ($C_1$-$C_4$)-alkyl;
S2 is ($C_1$-$C_4$)-alkyl or H;
where, if S1 and S2 are alkyl, $X^-$ in the resulting grouping [-$N^+$(S1 S2)- $X^-$] corresponds to a pharmacologically acceptable anion or trifluoroacetate;
B is a saturated or unsaturated five-, six- or seven-membered carbon ring which may be mono- or, independently of one another, polysubstituted by oxo, hydroxyl, ($C_1$-$C_4$)-alkoxy and ($C_1$-$C_4$)-alkyl;

and

R1, R2, R3, R4 and R5 are, independently of one another, H, OH, F, Cl, Br, I, CN, $NO_2$, amidino, -$CO_2$R(11), -CONR(11)R(12), -$SO_r$R(11), -$SO_s$NR(11)-R(12), ($C_1$-$C_4$)-alkyl, ($C_1$-$C_4$)-alkoxy, ($C_1$-$C_4$)-alkoxy-($C_1$-$C_4$)-alkyl, ($C_1$-$C_4$)-alkoxy-($C_1$-$C_4$)-alkyloxy, hydroxy-($C_1$-$C_4$)-alkyl, ($C_3$-$C_7$)-cycloalkoxy or phenyloxy,
where phenyl is unsubstituted or substituted by up to three substituents, which are independent of one another and selected from the group consisting of F, Cl, Br and methoxy;
amino, ($C_1$-$C_4$)-alkylamino, di-($C_1$-$C_4$)-alkylamino, amino-($C_1$-$C_4$)-alkyl, di-($C_1$-$C_4$)-alkylamino-($C_1$-$C_4$)-alkyl, ($C_1$-$C_4$)-alkylamino-($C_1$-$C_4$)-alkyl,
where some or all of the hydrogen atoms in the alkyl radicals may be substituted by fluorine;

R11 and R12 are, independently of one another, H or ($C_1$-$C_4$)-alkyl,
where some or all of the hydrogen atoms in the alkyl radicals may be substituted by fluorine;
r is 0, 1 or 2;
s is 1 or 2;

or

R1 and R2, R2 and R3, R3 and R4 or R4 and R5 in each case together are a group -O-$(CH_2)_n$-O-;

n is 1 or 2;

and
the radicals R1, R2, R3, R4 or R5 which remain in each case
are, independently of one another, H, OH, F, Cl, Br, I, CN, $NO_2$, amidino, -$CO_2$R(11), -CONR(11)R(12), -$SO_r$R(11), -$SO_s$NR(11)-R(12), ($C_1$-$C_4$)-alkyl, ($C_1$-$C_4$)-alkoxy, ($C_1$-$C_4$)-alkoxy-($C_1$-$C_4$)-alkyl, ($C_3$-$C_7$)-cycloalkoxy, hydroxy-($C_1$-$C_4$)-alkyl, amino, ($C_1$-$C_4$)-alkylamino, di-($C_1$-$C_4$)-alkylamino, amino-($C_1$-$C_4$)-alkyl, di-($C_1$-$C_4$)-alkylamino-($C_1$-$C_4$)-alkyl, ($C_1$-$C_4$)-alkylamino-($C_1$-$C_4$)-alkyl,
where some or all of the hydrogen atoms in the alkyl radicals may be substituted by fluorine;

R11 and R12 are, independently of one another, H or ($C_1$-$C_4$)-alkyl,
where some or all of the hydrogen atoms in the alkyl radicals may be substituted by fluorine;
r is 0, 1 or 2;
s is 1 or 2;

except for benzyl(octahydro-4,7-methanoinden-5-yl)amine,
and its pharmaceutically acceptable salts or trifluoroacetates.

**2.** A compound of the formula I or I a as claimed in claim 1 having exo-configured nitrogen and an endo-fused five- or six-membered ring of the formula I or having exo-configured nitrogen and an exo-fused five- or six-membered ring of the formula I a, in which:

A is ($C_1$-$C_2$)-alkylene
S1 is a free electron pair or methyl,
S2 is H;
B is a saturated or unsaturated five- or six-membered carbon ring;
R1, R2, R3, R4 and R5 are, independently of one another, H, amino, hydroxymethyl, OH, methoxy, F, Cl, Br or iodine;

or

R2 and R3 together are -O-$CH_2$-O-;

and
the remaining radicals R1, R4 and R5
are, independently of one another, H, OH, F, Cl, Br, I, CN, $NO_2$, ($C_1$-$C_2$)-alkoxy, amino, ($C_1$-$C_2$)-alkylamino or di-($C_1$-$C_2$)-alkylamino,
where some or all of the hydrogen atoms in the alkyl radicals may be substituted by fluorine;
and its pharmaceutically acceptable salts or trifluoroacetates.

**3.** A compound of the formula I or I a as claimed in claim 1 or 2 having exo-configured nitrogen and an endo-fused five- or six-membered ring of the formula I or having exo-configured nitrogen and an exo-fused five- or six-membered ring of the formula I a, in which:

A is ($C_1$-$C_2$)-alkylene;
S1 is a free electron pair;
S2 is H;
B is a saturated or unsaturated five- or six-membered carbon ring;
R1, R3 and R5 are hydrogen;

and R2 and R4
are, independently of one another, H, methoxy, F or Cl;
or

R2 and R3 together are -O-$CH_2$-O-;

and

R1, R4 and R5 are hydrogen;

and its pharmaceutically acceptable salts.

**4.** A compound of the formula I or I a as claimed in one or more of claims 1 to 3 having exo-configured nitrogen and an endo-fused five- or six-membered ring of the formula I or having exo-configured nitrogen and an exo-fused five-membered ring of the formula I a, the following compounds being concerned:

exo/endo-(3-chlorobenzyl)(octahydro-4,7-methanoinden-5-yl)amine,
exo/endo-(3-fluorobenzyl)(octahydro-4,7-methanoinden-5-yl)amine;
exo/endo-benzo[1,3]dioxol-5-ylmethyl(octahydro-4,7-methanoinden-5-yl)-amine,
exo/endo-(*rac*)-(3-methoxybenzyl)(octahydro-4,7-methanoinden-5-yl)-amine,
exo/endo-(+)-(3-methoxybenzyl)(octahydro-4,7-methanoinden-5-yl)amine,
exo/endo-(-)-(3-methoxybenzyl)(octahydro-4,7-methanoinden-5-yl)amine,
exo/endo-[1-(3-methoxyphenyl)ethyl](octahydro-4,7-methanoinden-5-yl)-amine,
exo/endo-(3-fluorobenzyl)(3a,4,5,6,7,7a-hexahydro-1H-4,7-methanoinden-5-yl)amine,
exo/endo-(3-fluorobenzyl)(3a,4,5,6,7,7a-hexahydro-3H-4,7-methanoinden-5-yl)amine,
exo/endo-(3a,4,5,6,7,7a-hexahydro-1H-4,7-methanoinden-5-yl)(3-methoxybenzyl)amine,

exo/endo-(3a,4,5,6,7,7a-hexahydro-3H-4,7-methanoinden-5-yl)(3-methoxybenzyl)amine,
exo/endo-(decahydro-1,4-methanonaphthalen-2-yl)(3-methoxybenzyl)-amine,
exo/endo-(3,5-difluorobenzyl)(octahydro-4,7-methanoinden-5-yl)amine,
exo/exo-(3-fluorobenzyl)(octahydro-4,7-methanoinden-5-yl)amine and
exo/exo-(3-methoxybenzyl)(octahydro-4,7-methanoinden-5-yl)amine
and its pharmaceutically acceptable salts or trifluoroacetates.

**5.** A compound of the formula I as claimed in one or more of claims 1 to 4, having exo-configured amine and an endo-fused five- or six-membered ring, the following compounds being concerned:

exo/endo-(3-chlorobenzyl)(octahydro-4,7-methanoinden-5-yl)amine,
exo/endo-(3-fluorobenzyl)(octahydro-4,7-methanoinden-5-yl)amine
exo/endo-(3-fluorobenzyl)(3a,4,5,6,7,7a-hexahydro-1H-4,7-methanoinden-5-yl)amine,
exo/endo-(3-fluorobenzyl)(3a,4,5,6,7,7a-hexahydro-3H-4,7-methanoinden-5-yl)amine,
exo/endo-benzo[1,3]dioxol-5-ylmethyl(octahydro-4,7-methanoinden-5-yl)-amine,
exo/endo-(*rac*)-(3-methoxybenzyl)(octahydro-4,7-methanoinden-5-yl)amine,
exo/endo-(+)-(3-methoxybenzyl)(octahydro-4,7-methanoinden-5-yl)amine,
exo/endo-(decahydro-1,4-methanonaphthalen-2-yl)(3-methoxybenzyl)-amine,
exo/endo-(-)-(3-methoxybenzyl)(octahydro-4,7-methanoinden-5-yl)amine and
exo/endo-(3,5-difluorobenzyl)(octahydro-4,7-methanoinden-5-yl)amine,
and its pharmaceutically acceptable salts or trifluoroacetates.

**6.** A process for preparing a compound of the formula I or I a as claimed in claim 1, which comprises

a) reacting a compound of the formula II or II a

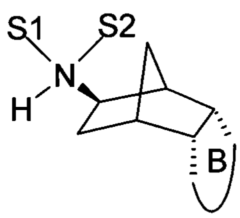 II

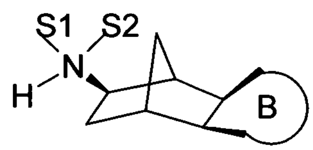

II a

with a compound of the formula III in the presence of suitable reducing agents and optionally also Lewis acids directly to give compounds of the formula I or I a

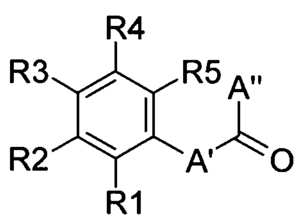 III

in which S1, S2, B, R1, R2, R3, R4 and R5 are as defined above, while independently of one another A' is a bond or ($C_1$-$C_3$)-alkyl and A" is H or ($C_1$-$C_3$)-alkyl and A' and A" together with the carbon atom of the carbonyl group represent the same number of carbon atoms as A;
or
b) isolating the intermediate of the formula IV or IV a

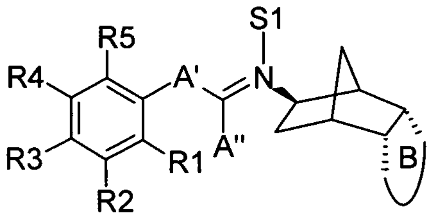 IV 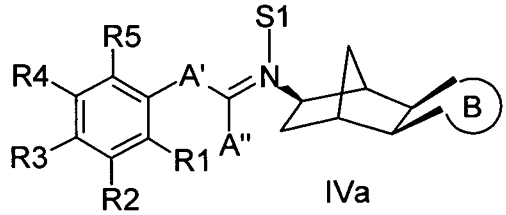 IVa

formed from compounds of the formulae II or II a and III, in which, if S1 is ($C_1$-$C_4$)-alkyl, an onium nitrogen is formed which is associated with a counterion, such as, for example, chloride or tosylate,
and then converting the intermediate with suitable reducing agents into the compounds of the formula I or Ia,
or
c) reacting a compound of the formula II or II a with an alkylating agent of the formula V

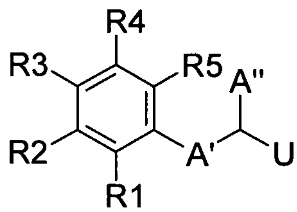 V

in which U is a nucleophilically substitutable group - such as chlorine, bromine and iodine and also mesylate, tosylate or triflate - and the other radicals are as defined above, but where the carbon atom to which U is attached corresponds to the carbon atom of the carbonyl group,
preferably in the presence of non-nucleophilic bases, such as diisopropylethylamine,
or
d) reducing carboxamides of the formula VI or VI a

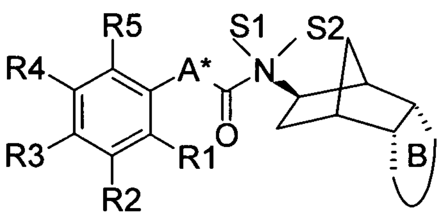 VI 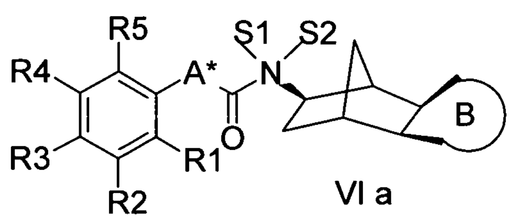 VI a

in which A* is a bond or ($C_1$-$C_3$)-alkyl and the other radicals are as defined above,
to give the corresponding amines;
or
e) mono- or dialkylating compounds of the formula I or Ia in which S1 is a free electron pair and S2 is hydrogen, with alkylating agents of the formula VII

S*—U VII

in which S* is ($C_1$-$C_4$)-alkyl and U is as defined above, thus obtaining tertiary amines or quaternary ammonium salts;
or
f) reacting a dicyclopentadienylplatinum complex of the formula VIII

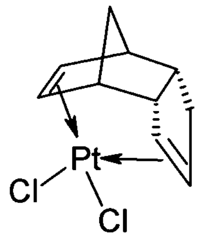

VIII

with amines of the type of the formula IX

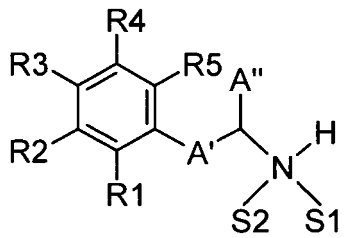

IX

in which S1, S2, R1, R2, R3, R4 and R5 are as defined above, while independently of one another A' is a bond or ($C_1$-$C_3$)-alkyl and A" is H or ($C_1$-$C_3$)-alkyl and A' and A" together with the carbon atom to which the nitrogen atom is attached represent the same number of carbon atoms as A,

and then reducing the intermediate formed to give compounds of the formula I;
optionally followed by conversion into the pharmaceutically acceptable salt or trifluoroacetate.

**7.** The use of a compound I or I a as claimed in claim 1 for the production of a medicament for the treatment or prophylaxis of disorders of the respiratory drive.

**8.** The use of a compound I or I a as claimed in claim 1 for the production of a medicament for the treatment or prophylaxis of respiratory disorders, in particular sleep-related respiratory disorders, such as sleep apneas.

**9.** The use of a compound I or I a as claimed in claim 1 for the production of a medicament for the treatment or prophylaxis of snoring.

**10.** The use of a compound I or I a as claimed in claim 1 for the production of a medicament for the treatment or prophylaxis of acute or chronic renal disorders, in particular acute kidney failure and chronic kidney failure.

**11.** The use of a compound I or I a as claimed in claim 1 for the production of a medicament for the treatment or prophylaxis of impaired intestinal function.

**12.** The use of a compound I or Ia as claimed in claim 1 for the production of a medicament for the treatment or prophylaxis of impaired gallbladder function.

**13.** The use of a compound I or I a as claimed in claim 1 for the production of a medicament for the treatment or prophylaxis of ischemic states of the peripheral and central nervous system and of stroke.

**14.** The use of a compound I or I a as claimed in claim 1 for the production of a medicament for the treatment or prophylaxis of ischemic states of peripheral organs and limbs.

**15.** The use of a compound I or I a as claimed in claim 1 for the production of a medicament for the treatment of states of shock.

**16.** The use of a compound I or I a as claimed in claim 1 for the production of a medicament for use in surgical interventions and organ transplants.

**17.** The use of a compound I or I a as claimed in claim 1 for the production of a medicament for the preservation and storage of transplants for surgical interventions.

**18.** The use of a compound I or I a as claimed in claim 1 for the production of a medicament for the treatment of diseases whose primary or secondary cause is cell proliferation.

**19.** The use of a compound I or I a as claimed in claim 1 for the production of a medicament for the treatment or prophylaxis of an impaired lipid metabolism.

**20.** The use of a compound I or I a as claimed in claim 1 for the production of a medicament for the treatment or prophylaxis of infestation by ectoparasites.

**21.** A medicine, comprising an effective amount of a compound I or Ia as claimed in one or more of claims 1 to 5.

## Revendications

**1.** Dérivés norbonylamino substitués à atome d'azote en configuration exo et cycle pentagonal, hexagonal ou heptagonal endo-condensé de formule I ou à atome d'azote en configuration exo et cycle pentagonal, hexagonal ou heptagonal exo-condensé de formule la

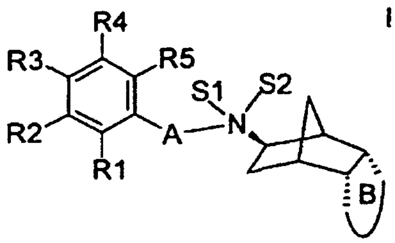

I

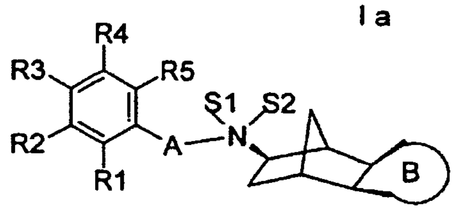

I a

formules dans lesquelles :

A représente un groupe alkylène en $C_1$-$C_4$ ;
S1 représente une paire d'électrons libres ou un groupe alkyle en $C_1$-$C_4$ ;
S2 représente un groupe alkyle en $C_1$-$C_4$ ou H ;
lorsque S1 et S2 représentent un groupe alkyle, $X^-$ dans le groupement [-$N^+$(S1S2)-$X^-$] résultant correspondant au trifluoroacétate ou à un anion pharmaceutiquement acceptable ;
B représente un cycle carbocyclique pentagonal, hexagonal ou heptagonal saturé ou insaturé qui peut porter un ou plusieurs substituants, indépendamment les uns des autres, oxo, hydroxy, alcoxy en $C_1$-$C_4$ et alkyle en $C_1$-$C_4$ ;

et

R1, R2, R3, R4 et R5 représentent, indépendamment les uns des autres, H, OH, F, Cl, Br, I, CN, $NO_2$, un groupe amidino, -$CO_2$R(11), -CONR(11)R(12), -$SO_r$R(11), -$SO_s$NR(11)R(12), alkyle en $C_1$-$C_4$, alcoxy en $C_1$-$C_4$, alcoxy($C_1$-$C_4$)-alkyle($C_1$-$C_4$), alcoxy($C_1$-$C_4$)-alkyloxy($C_1$-$C_4$), hydroxyalkyle($C_1$-$C_4$), cycloalalcoxy en $C_3$-$C_7$ ou phénoxy,
le radical phényle étant non substitué ou portant jusqu'à trois substituants qui sont choisis, chacun indépendamment, dans l'ensemble consistant en F, Cl, Br et méthoxy ;
amino, alkyl($C_1$-$C_4$)amino, dialkyl($C_1$-$C_4$)amino, aminoalkyle en $C_1$-$C_4$, dialkyl($C_1$-$C_4$)amino-alkyle($C_1$-$C_4$), alkyl ($C_1$-$C_4$)amino-alkyle($C_1$-$C_4$),
les atomes d'hydrogène dans les radicaux alkyle pouvant être partiellement ou totalement remplacés par le fluor ;

R11 et R12 représentent, indépendamment l'un de l'autre, H ou un groupe alkyle en $C_1$-$C_4$,
les atomes d'hydrogène dans les radicaux alkyle pouvant être partiellement ou totalement remplacés par le fluor ;

r est 0, 1 ou 2 ;
s est 1 ou 2 ;

ou

R1 et R2, R2 et R3, R3 et R4 ou bien R4 et R5 forment respectivement ensemble un groupe $-O-(CH_2)_n-O-$,

n est 1 ou 2 ;

et
les radicaux R1, R2, R3 R4 ou R5 restants dans chaque cas représentent, indépendamment les uns des autres, H, OH, F, Cl, Br, I, CN, $NO_2$, un groupe amidino, $-CO_2R(11)$, -CONR(11)R(12), $-SO_rR(11)$, $-SO_sNR(11)R(12)$, alkyle en $C_1$-$C_4$, alcoxy en $C_1$-$C_4$, alcoxy($C_1$-$C_4$)-alkyle($C_1$-$C_4$), cycloalcoxy en $C_3$-$C_7$, hydroxyalkyle($C_1$-$C_4$), amino, alkyl ($C_1$-$C_4$)amino, dialkyl($C_1$-$C_4$)amino, aminoalkyle en $C_1$-$C_4$, dialkyl($C_1$-$C_4$)amino-alkyle($C_1$-$C_4$), alkyl($C_1$-$C_4$)aminoalkyle($C_1$-$C_4$),
les atomes d'hydrogène dans les radicaux alkyle pouvant être partiellement ou totalement remplacés par le fluor ;

R11 et R12 représentent, indépendamment l'un de l'autre, H ou un groupe alkyle en $C_1$-$C_4$,
les atomes d'hydrogène dans les radicaux alkyle pouvant être partiellement ou totalement remplacés par le fluor ;
r est 0, 1 ou 2 ;
s est 1 ou 2 ;

à l'exclusion de la benzyl-(octahydro-4,7-méthano-indén-5-yl)amine, ainsi que leurs trifluoroacétates ou sels pharmaceutiquement acceptables.

**2.** Composés de formule I ou la selon la revendication 1, à atome d'azote en configuration exo et cycle pentagonal ou hexagonal endo-condensé de formule I ou à atome d'azote en configuration exo et cycle pentagonal ou hexagonal exo-condensé de formule la, dans lesquels :

A représente un groupe alkylène en $C_1$-$C_2$ ;
S1 représente une paire d'électrons libres ou le groupe méthyle ;
S2 représente H ;
B représente un cycle carbocyclique pentagonal ou hexagonal saturé ou insaturé ;
R1, R2, R3, R4 et R5 représentent, indépendamment les uns des autres, H, un groupe amino, hydroxyméthyle, OH, méthoxy, F, Cl, Br ou un atome d'iode ;

ou

R2 et R3 forment ensemble un groupe $-O-CH_2-O-$ ;

et
les radicaux R1, R4 et R5 restants
représentent, chacun indépendamment, H, OH, F, Cl, Br, I, CN, $NO_2$, un groupe alcoxy en $C_1$-$C_2$, amino, alkyl ($C_1$-$C_2$)amino ou dialkyl($C_1$-$C_2$)amino,
les atomes d'hydrogène dans les radicaux alkyle pouvant être partiellement ou totalement remplacés par le fluor ;
ainsi que leurs trifluoroacétates ou sels pharmaceutiquement acceptables.

**3.** Composés de formule I ou la selon les revendications 1 et 2, à atome d'azote en configuration exo et cycle pentagonal ou hexagonal endo-condensé de formule I ou à atome d'azote en configuration exo et cycle pentagonal ou hexagonal exo-condensé de formule la, dans lesquels :

A représente un groupe alkylène en $C_1$-$C_2$ ;
S1 représente une paire d'électrons libres ;
S2 représente H ;
B représente un cycle carbocyclique pentagonal ou hexagonal saturé ou insaturé ;
R1, R3 et R5 représentent un atome d'hydrogène ;
et
R2 et R4 représentent, indépendamment l'un de l'autre, H, F ou Cl ou le groupe méthoxy ;

ou

R2 et R3 forment ensemble un groupe $-O-CH_2-O-$ ;

et
les radicaux R1, R4 et R5
représentent un atome d'hydrogène ;
ainsi que leurs sels pharmaceutiquement acceptables.

**4.** Composés de formule I ou la selon une ou plusieurs des revendications 1 à 3, à atome d'azote en configuration exo et cycle pentagonal ou hexagonal endo-condensé de formule I ou à atome d'azote en configuration exo et cycle pentagonal exo-condensé de formule la, **caractérisés en ce qu'**il s'agit des composés suivants :

exo/endo-(3-chlorobenzyl)-(octahydro-4,7-méthano-indén-5-yl)amine,
exo/endo-(3-fluorobenzyl)-(octahydro-4,7-méthano-indén-5-yl)amine,
exo/endo-benzo[1,3]dioxol-5-ylméthyl-(octahydro-4,7-méthano-indén-5-yl)amine,
exo/endo-(*rac*)-(3-méthoxy-benzyl)-(octahydro-4,7-méthano-indén-5-yl)amine,
exo/endo-(+)-(3-méthoxy-benzyl)-(octahydro-4,7-méthano-indén-5-yl)amine,
exo/endo-(-)-(3-méthoxy-benzyl)-(octahydro-4,7-méthano-indén-5-yl)amine,
exo/endo-[1-(3-méthoxy-phényl)-éthyl]-(octahydro-4,7-méthano-indén-5-yl)amine,
exo/endo-(3-fluorobenzyl)-(3a,4,5,6,7,7a-hexahydro-1 H-4,7-méthano-indén-5-yl)amine,
exo/endo-(3-fluorobenzyl)-(3a,4,5,6,7,7a-hexahydro-3H-4,7-méthano-indén-5-yl)amine,
exo/endo-(3a,4,5,6,7,7a-hexahydro-1H-4,7-méthano-indén-5-yl)-(3-méthoxybenzyl)amine,
exo/endo-(3a,4,5,6,7,7a-hexahydro-3H-4,7-méthano-indén-5-yl)-(3-méthoxybenzyl)amine,
exo/endo-(décahydro-1,4-méthano-naphtalén-2-yl)-(3-méthoxybenzyl)amine,
exo/endo-(3,5-difluorobenzyl)-(octahydro-4,7-méthano-indén-5-yl)amine,
exo/exo-(3-fluorobenzyl)-(octahydro-4,7-méthano-indén-5-yl)amine et
exo/exo-(3-méthoxybenzyl)-(octahydro-4,7-méthano-indén-5-yl)amine,
ainsi que leurs trifluoroacétates et sels pharmaceutiquement acceptables.

**5.** Composés de formule I selon une ou plusieurs des revendications 1 à 4, à amine en position exo et cycle pentagonal ou hexagonal endo-condensé, qui sont les suivants :

exo/endo-(3-chlorobenzyl)-(octahydro-4,7-méthano-indén-5-yl)amine,
exo/endo-(3-fluorobenzyl)-(octahydro-4,7-méthano-indén-5-yl)amine,
exo/endo-(3-fluorobenzyl)-(3a,4,5,6,7,7a-hexahydro-1H-4,7-méthano-indén-5-yl)amine,
exo/endo-(3-fluorobenzyl)-(3a,4,5,6,7,7a-hexahydro-3H-4,7-méthano-indén-5-yl)amine,
exo/endo-benzo[1,3]dioxol-5-ylméthyl-(octahydro-4,7-méthano-indén-5-yl)amine,
exo/endo-(*rac*)-(3-méthoxy-benzyl)-(octahydro-4,7-méthano-indén-5-yl)amine,
exo/endo-(+)-(3-méthoxy-benzyl)-(octahydro-4,7-méthano-indén-5-yl)amine,
exo/endo-(décahydro-1,4-méthano-naphtalén-2-yl)-(3-méthoxybenzyl)amine,
exo/endo-(-)-(3-méthoxy-benzyl)-(octahydro-4,7-méthano-indén-5-yl)amine et
exo/endo-(3,5-difluorobenzyl)-(octahydro-4,7-méthano-indén-5-yl)amine,
ainsi que leurs trifluoroacétates et sels pharmaceutiquement acceptables.

**6.** Procédé pour la préparation des composés de formule I ou la selon la revendication 1, **caractérisé en ce que**

a) on fait réagir un composé de formule II ou IIa

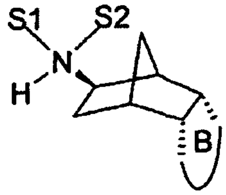
II

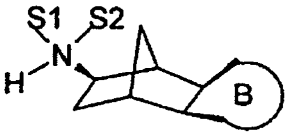
II a

avec un composé de formule III

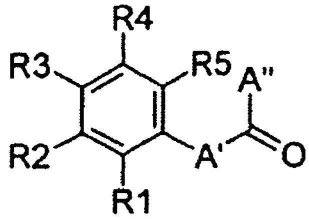

III

en présence de réducteurs appropriés et éventuellement aussi d'acides de Lewis, pour aboutir directement aux composés de formule I ou la,

dans lesquels S1, S2, B, R1, R2, R3, R4 et R5 ont les significations données plus haut, tandis que, indépendamment l'un de l'autre, A' correspond à une liaison ou à un groupe alkyle en $C_1$-$C_3$ et A" correspond à H ou à un groupe alkyle en $C_1$-$C_3$ ou A' et A" représentent ensemble, avec l'atome de carbone du groupe carbonyle, autant d'atomes de carbone que A ;

ou

b) on isole le composé intermédiaire, formé à partir de composés de formules II ou IIa et III, de formule IV ou IVa,

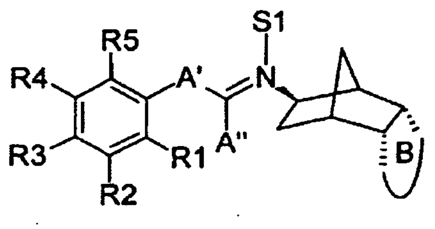

IV

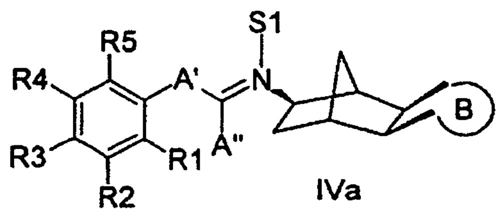

IVa

formules dans lesquelles lorsque S1 correspond à un groupe alkyle en $C_1$-$C_4$, il se forme un atome d'azote en fonction onium auquel est affecté un ion opposé, comme par exemple chlorure ou tosylate,

et on le convertit ensuite, à l'aide de réducteurs appropriés, en les composés de formule I ou la,

ou

c) on fait réagir, de préférence en présence de bases non nucléophiles telles que la diisopropyléthylamine, un composé de formule II ou IIa avec un agent d'alkylation de formule V,

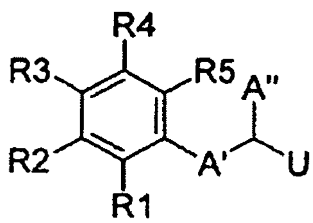

V

dans laquelle U représente un groupe pouvant être remplacé par substitution nucléophile - tel que chloro, bromo ou iodo ou bien mésylate, tosylate ou triflate - et les autres radicaux sont définis comme décrit plus haut, mais en ce cas l'atome de carbone du groupe carbonyle correspond à l'atome de carbone auquel est lié U,

ou

d) on réduit en les amines correspondantes des carboxamides de formule VI ou VIa

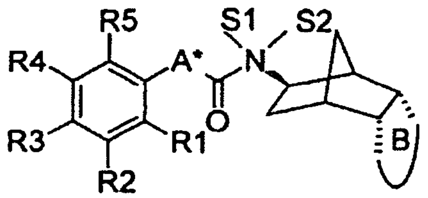

VI

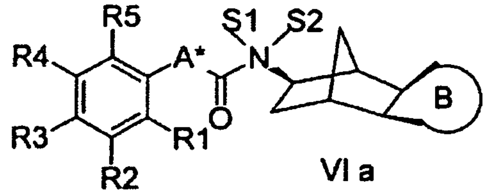

VI a

formules dans lesquelles A* correspond à une liaison ou à un groupe alkyle en $C_1$-$C_3$ et les autres radicaux sont définis comme décrit plus haut ;
ou
e) on soumet des composés de formule I ou Ia, dans lesquels S1 correspond à une paire d'électrons libres et S2 correspond représente un atome d'hydrogène, à une mono- ou dialkylation avec des agents d'alkylation de formule VII,

S*—U VII

dans laquelle S* représente un groupe alkyle en $C_1$-$C_4$ et U a la signification décrite plus haut, de sorte que des amines tertiaires ou des sels d'ammonium quaternaire résultent de cette réaction ;
ou
f) on fait réagir un complexe de dicyclopentadiénylplatine de formule VIII

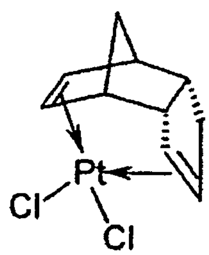

VIII

avec des amines du type de formule IX,

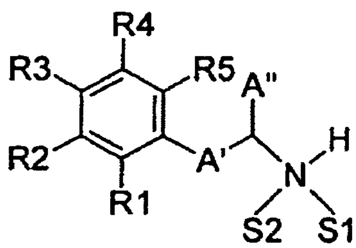

IX

dans laquelle S1, S2, R2, R3, R4 et R5 ont les significations données plus haut, tandis que, indépendamment l'un de l'autre, A' correspond à une liaison ou à un groupe alkyle en $C_1$-$C_3$ et A" correspond à H ou à un groupe alkyle en $C_1$-$C_3$ ou bien A' et A" représentent ensemble, avec l'atome de carbone auquel est lié l'atome d'azote, autant d'atomes de carbone que A,

et on réduit ensuite le produit intermédiaire formé en composés de formule I
et **en ce qu'**éventuellement on les convertit en le trifluoroacétate ou en un sel pharmaceutiquement acceptable.

7. Utilisation d'un composé I ou Ia selon la revendication 1, pour la fabrication d'un médicament destiné au traitement ou à la prophylaxie de troubles de l'impulsion respiratoire.

8. Utilisation d'un composé I ou Ia selon la revendication 1, pour la fabrication d'un médicament destiné au traitement

ou à la prophylaxie de troubles respiratoires, en particulier de troubles respiratoires dus au sommeil, tels que les apnées du sommeil.

**9.** Utilisation d'un composé I ou Ia selon la revendication 1, pour la fabrication d'un médicament destiné au traitement ou à la prophylaxie du ronflement.

**10.** Utilisation d'un composé I ou Ia selon la revendication 1, pour la fabrication d'un médicament destiné au traitement ou à la prophylaxie de néphropathies aiguës et chroniques, en particulier de l'insuffisance rénale aiguë et de l'insuffisance rénale chronique.

**11.** Utilisation d'un composé I ou Ia selon la revendication 1, pour la fabrication d'un médicament destiné au traitement ou à la prophylaxie de troubles de la fonction intestinale.

**12.** Utilisation d'un composé I ou Ia selon la revendication 1, pour la fabrication d'un médicament destiné au traitement ou à la prophylaxie de la fonction biliaire.

**13.** Utilisation d'un composé I ou Ia selon la revendication 1, pour la fabrication d'un médicament destiné au traitement ou à la prophylaxie d'états ischémiques du système nerveux central et périphérique et de l'accident vasculaire cérébral.

**14.** Utilisation d'un composé I ou Ia selon la revendication 1, pour la fabrication d'un médicament destiné au traitement ou à la prophylaxie d'états ischémiques des organes périphériques et des membres.

**15.** Utilisation d'un composé I ou Ia selon la revendication 1, pour la fabrication d'un médicament destiné au traitement d'états de choc.

**16.** Utilisation d'un composé I ou Ia selon la revendication 1, pour la fabrication d'un médicament destiné à l'utilisation dans des opérations chirurgicales et des transplantations d'organes.

**17.** Utilisation d'un composé I ou Ia selon la revendication 1, pour la fabrication d'un médicament destiné à la conservation et au stockage de transplants pour des interventions chirurgicales.

**18.** Utilisation d'un composé I ou Ia selon la revendication 1, pour la fabrication d'un médicament destiné au traitement ou à la prophylaxie de maladies dans lesquelles la prolifération cellulaire représente une cause primaire ou secondaire.

**19.** Utilisation d'un composé I ou Ia selon la revendication 1, pour la fabrication d'un médicament destiné au traitement ou à la prophylaxie de troubles du métabolisme lipidique.

**20.** Utilisation d'un composé I ou Ia selon la revendication 1, pour la fabrication d'un médicament destiné au traitement ou à la prophylaxie de l'attaque par des ectoparasites.

**21.** Médicament, contenant une quantité efficace d'un composé I ou Ia selon une ou plusieurs des revendications 1 à 5.